# EUROPEAN PATENT APPLICATION

(11) **EP 4 199 686 A1**
(43) Date of publication of application: **21.06.2023**
(21) Application number: 22208522.7
(22) Date of filing: 21.11.2022
(51) Int. Cl.: H10K 85/60, H10K 50/19, H10K 50/13, H10K 59/32, H10K 50/16, H10K 50/18

(54) **ORGANIC LIGHT EMITTING DIODE AND ORGANIC LIGHT EMITTING DEVICE INCLUDING THE SAME**

(30) Priority: 14.12.2021 KR 20210178382
(71) Applicant: LG Display Co., Ltd., SEOUL, 07336 (KR); Jinwoong Industrial Co., Ltd., Yangju-si, Gyeonggi-do 11417 (KR)
(72) Inventor: KIM, Shin-Han, 10845 Paju-si (KR); HYUNG, Min-Surk, 10845 Paju-si (KR); SEO, Jeong-Dae, 10845 Paju-si (KR); JEONG, Eun-Been, 10845 Paju-si (KR); LEE, So-Hee, 10845 Paju-si (KR); JANG, Yu-Jeong, 10845 Paju-si (KR); LEE, Keum-Hee, 10845 Paju-si (KR); KIM, Moon-Bae, 10845 Paju-si (KR); NA, Seok-Ho, 10845 Paju-si (KR)
(74) Representative: Ter Meer Steinmeister & Partner

(57) **Abstract**

An organic light emitting diode includes a first electrode; a second electrode facing the first electrode; a first emitting material layer between the first and second electrodes; and a first intermediate organic layer between the first emitting material layer and the second electrode and including a first organic layer and a second organic layer between the first organic layer and the second electrode, wherein the first organic layer includes a first compound represented by Formula.

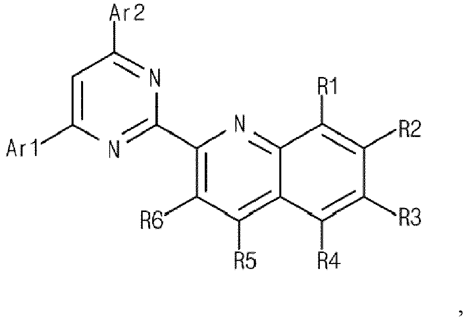

## Description

### BACKGROUND

### TECHNICAL FIELD

The present disclosure relates to an organic light emitting diode, and more particularly, to an organic light emitting diode having low driving voltage, high emitting efficiency and long lifespan and an organic light emitting device including the organic light emitting diode.

### DISCUSSION OF THE RELATED ART

Recently, requirement for flat panel display devices having small occupied area is increased. Among the flat panel display devices, a technology of an organic light emitting display device, which includes an organic light emitting diode (OLED) and may be called to as an organic electroluminescent device, is rapidly developed.

The OLED emits light by injecting electrons from a cathode as an electron injection electrode and holes from an anode as a hole injection electrode into an emitting material layer, combining the electrons with the holes, generating an exciton, and transforming the exciton from an excited state to a ground state.

The related art OLED has problem of increase of power consumption and decrease of lifespan by high driving voltage.

### SUMMARY

Accordingly, embodiments of the present disclosure are directed to an OLED and an organic light emitting device that substantially obviate one or more of the problems associated with the limitations and disadvantages of the related art.

An object of the present disclosure is to provide an OLED and an organic light emitting device having low driving voltage, high emitting efficiency and long lifespan.

Additional features and aspects will be set forth in the description that follows, and in part will be apparent from the description, or may be learned by practice of the present disclosure concepts provided herein. Other features and aspects of the present disclosure concepts may be realized and attained by the structure particularly pointed out in the written description, or derivable therefrom, and the claims hereof as well as the appended drawings.

To achieve these and other advantages and in accordance with objects of the disclosure, as described herein, an aspect of the present disclosure is an organic light emitting diode including a first electrode; a second electrode facing the first electrode; a first emitting material layer between the first and second electrodes; and a first intermediate organic layer between the first emitting material layer and the second electrode and including a first organic layer and a second organic layer between the first organic layer and the second electrode, wherein the first organic layer includes a first compound represented by Formula 1-1: [Formula 1-1] 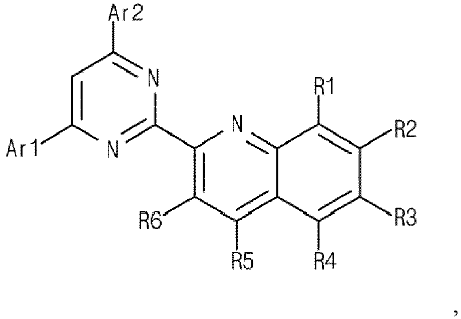 wherein each of Ar1 and Ar2 is independently selected form the group consisting of a substituted or unsubstituted C6 to C50 aryl group and a substituted or unsubstituted C5 to C50 heteroaryl group, and wherein each of R1 to R6 is independently selected form the group consisting of hydrogen, deuterium, a substituted or unsubstituted C1 to C10 alkyl group, a substituted or unsubstituted C6 to C30 aryl group and a substituted or unsubstituted C5 to C30 heteroaryl group.

In another aspect of the present disclosure, an organic light emitting device includes a substrate; and an organic light emitting diode positioned over the substrate, the organic light emitting diode being according to the invention. represented by Formula 1-1: [Formula 1-1]

It is to be understood that both the foregoing general description and the following detailed description of the present disclosure are merely by way of example and are intended to provide further explanation of the inventive concepts as claimed.

### BRIEF DESCRIPTION OF THE DRAWINGS

The accompanying drawings, which are included to provide a further understanding of the present disclosure and are incorporated in and constitute a part of this application, illustrate embodiments of the present disclosure and together with the description serve to explain principles of the present disclosure.
FIG. 1 is a schematic circuit diagram of an organic light emitting display device of the present disclosure.
FIG. 2 is a schematic cross-sectional view of an organic light emitting display device according to a first embodiment of the present disclosure.
FIG. 3 is a schematic cross-sectional view of an OLED according to a second embodiment of the present disclosure.
FIG. 4 is a schematic cross-sectional view of an OLED according to a third embodiment of the present disclosure.
FIG. 5 is a schematic cross-sectional view of an organic light emitting display device according to a fourth embodiment of the present disclosure.
FIG. 6 is a schematic cross-sectional view of an OLED according to a fifth embodiment of the present disclosure.
FIG. 7 is a schematic cross-sectional view of an OLED according to a sixth embodiment of the present disclosure.

### DETAILED DESCRIPTION

Reference will now be made in detail to some of the examples and embodiments of the disclosure illustrated in the accompanying drawings. Wherever possible, the same reference numbers will be used throughout the drawings to refer to the same or like parts.

The present disclosure provides an OLED and an organic light emitting device. For example, the organic light emitting device may be an organic light emitting display device or an organic light emitting lighting device. The explanation below is focused on the organic light emitting display device including the OLED.

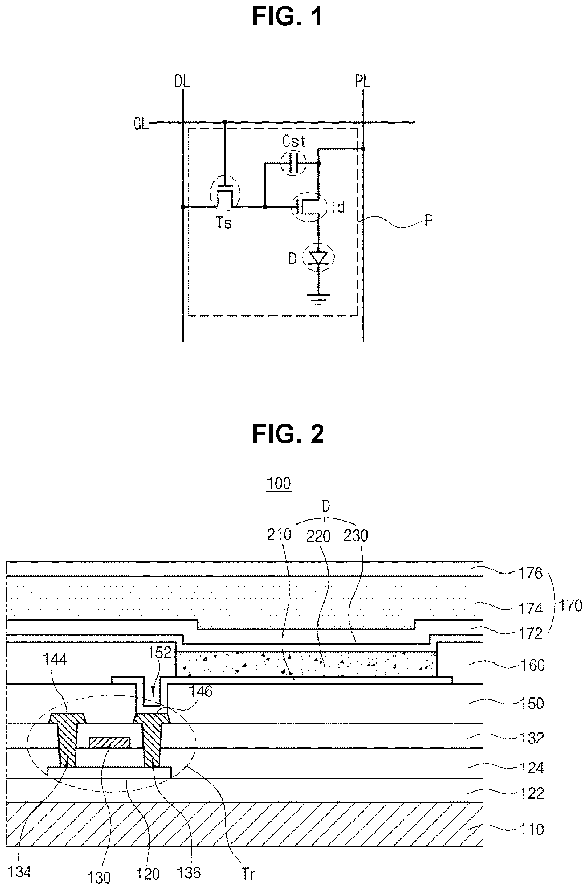

FIG. 1 is a schematic circuit diagram of an organic light emitting display device of the present disclosure.

As shown in FIG. 1, an organic light emitting display device includes a gate line GL, a data line DL, a power line PL, a switching thin film transistor TFT Ts, a driving TFT Td, a storage capacitor Cst, and an OLED D. The gate line GL and the data line DL cross each other to define a pixel region P. The pixel region may include a red pixel region, a green pixel region and a blue pixel region.

The switching TFT Ts is connected to the gate line GL and the data line DL, and the driving TFT Td and the storage capacitor Cst are connected to the switching TFT Ts and the power line PL. The OLED D is connected to the driving TFT Td.

In the organic light emitting display device, when the switching TFT Ts is turned on by a gate signal applied through the gate line GL, a data signal from the data line DL is applied to the gate electrode of the driving TFT Td and an electrode of the storage capacitor Cst.

When the driving TFT Td is turned on by the data signal, an electric current is supplied to the OLED D from the power line PL. As a result, the OLED D emits light. In this case, when the driving TFT Td is turned on, a level of an electric current applied from the power line PL to the OLED D is determined such that the OLED D can produce a gray scale.

The storage capacitor Cst serves to maintain the voltage of the gate electrode of the driving TFT Td when the switching TFT Ts is turned off. Accordingly, even if the switching TFT Ts is turned off, a level of an electric current applied from the power line PL to the OLED D is maintained to next frame.

As a result, the organic light emitting display device displays a desired image.

FIG. 2 is a schematic cross-sectional view of an organic light emitting display device according to a first embodiment of the present disclosure.

As shown in FIG. 2, the organic light emitting display device 100 includes a substrate 110, a TFT Tr on or over the substrate 110, a planarization layer 150 covering the TFT Tr and an OLED D on the planarization layer 150 and connected to the TFT Tr.

The substrate 110 may be a glass substrate or a flexible substrate. For example, the flexible substrate may be one of a polyimide (PI) substrate, a polyethersulfone (PES) substrate, a polyethylenenaphthalate (PEN) substrate, a polyethylene terephthalate (PET) substrate and a polycarbonate (PC) substrate.

A buffer layer 122 is formed on the substrate, and the TFT Tr is formed on the buffer layer 122. The buffer layer 122 may be omitted. For example, the buffer layer 122 may be formed of an inorganic insulating material, e.g., silicon oxide or silicon nitride.

A semiconductor layer 120 is formed on the buffer layer 122. The semiconductor layer 120 may include an oxide semiconductor material or polycrystalline silicon.

When the semiconductor layer 120 includes the oxide semiconductor material, a light-shielding pattern (not shown) may be formed under the semiconductor layer 120. The light to the semiconductor layer 120 is shielded or blocked by the light-shielding pattern such that thermal degradation of the semiconductor layer 120 can be prevented. On the other hand, when the semiconductor layer 120 includes polycrystalline silicon, impurities may be doped into both sides of the semiconductor layer 120.

A gate insulating layer 124 of an insulating material is formed on the semiconductor layer 120. The gate insulating layer 124 may be formed of an inorganic insulating material such as silicon oxide or silicon nitride.

A gate electrode 130, which is formed of a conductive material, e.g., metal, is formed on the gate insulating layer 124 to correspond to a center of the semiconductor layer 120. In FIG. 2, the gate insulating layer 124 is formed on an entire surface of the substrate 110. Alternatively, the gate insulating layer 124 may be patterned to have the same shape as the gate electrode 130.

An interlayer insulating layer 132 of an insulating material is formed on the gate electrode 130 and over an entire surface of the substrate 110. The interlayer insulating layer 132 may be formed of an inorganic insulating material, e.g., silicon oxide or silicon nitride, or an organic insulating material, e.g., benzocyclobutene or photo-acryl.

The interlayer insulating layer 132 includes first and second contact holes 134 and 136 exposing both sides of the semiconductor layer 120. The first and second contact holes 134 and 136 are positioned at both sides of the gate electrode 130 to be spaced apart from the gate electrode 130.

The first and second contact holes 134 and 136 are formed through the gate insulating layer 124. Alternatively, when the gate insulating layer 124 is patterned to have the same shape as the gate electrode 130, the first and second contact holes 134 and 136 is formed only through the interlayer insulating layer 132.

A source electrode 144 and a drain electrode 146, which are formed of a conductive material, e.g., metal, are formed on the interlayer insulating layer 132.

The source electrode 144 and the drain electrode 146 are spaced apart from each other with respect to the gate electrode 130 and respectively contact both sides of the semiconductor layer 120 through the first and second contact holes 134 and 136.

The semiconductor layer 120, the gate electrode 130, the source electrode 144 and the drain electrode 146 constitute the TFT Tr. The TFT Tr serves as a driving element. Namely, the TFT Tr is the driving TFT Td (of FIG. 1).

In the TFT Tr, the gate electrode 130, the source electrode 144, and the drain electrode 146 are positioned over the semiconductor layer 120. Namely, the TFT Tr has a coplanar structure.

Alternatively, in the TFT Tr, the gate electrode may be positioned under the semiconductor layer, and the source and drain electrodes may be positioned over the semiconductor layer such that the TFT Tr may have an inverted staggered structure. In this instance, the semiconductor layer may include amorphous silicon.

Although not shown, the gate line and the data line cross each other to define the pixel region, and the switching TFT is formed to be connected to the gate and data lines. The switching TFT is connected to the TFT Tr as the driving element. In addition, the power line, which may be formed to be parallel to and spaced apart from one of the gate and data lines, and the storage capacitor for maintaining the voltage of the gate electrode of the TFT Tr in one frame may be further formed.

A planarization layer 150 is formed on an entire surface of the substrate 110 to cover the source and drain electrodes 144 and 146. The planarization layer 150 provides a flat top surface and has a drain contact hole 152 exposing the drain electrode 146 of the TFT Tr.

The OLED D is disposed on the planarization layer 150 and includes a first electrode 210, which is connected to the drain electrode 146 of the TFT Tr, an light emitting layer 220 and a second electrode 230. The light emitting layer 220 and the second electrode 230 are sequentially stacked on the first electrode 210. The OLED D is positioned in each of red, green and blue pixel regions and respectively emits the red, green and blue light.

The first electrode 210 is separately formed in each pixel region. The first electrode 210 may be an anode and may include a transparent conductive oxide material layer, which may be formed of a conductive material, e.g., a transparent conductive oxide (TCO), having a relatively high work function. For example, the first electrode 210 may be formed of one of indium-tin-oxide (ITO), indium-zinc-oxide (IZO), indium-tin-zinc-oxide (ITZO), tin oxide (SnO), zinc oxide (ZnO), indium-copper-oxide (ICO) and aluminum-zinc-oxide (Al:ZnO, AZO).

When the organic light emitting display device 100 is operated in a bottom-emission type, the first electrode 210 may have a single-layered structure of the transparent conductive oxide material layer. When the organic light emitting display device 100 is operated in a top-emission type, the first electrode 210 may further include a reflection electrode or a reflection layer. For example, the reflection electrode or the reflection layer may be formed of silver (Ag) or aluminum-palladium-copper (APC) alloy. In the top-emission type organic light emitting display device 100, the first electrode 210 may have a triple-layered structure of ITO/Ag/ITO or ITO/APC/ITO.

In addition, a bank layer 160 is formed on the planarization layer 150 to cover an edge of the first electrode 210. Namely, the bank layer 160 is positioned at a boundary of the pixel region and exposes a center of the first electrode 210 in the pixel region.

The light emitting layer 220 as an emitting unit is formed on the first electrode 210. The light emitting layer 220 includes an emitting material layer (EML) and an intermediate organic layer. For example, the intermediate organic layer may has a double-layered structure including a first organic layer being a hole blocking layer (HBL) and a second organic layer being an electron transporting layer (ETL). In addition, the light emitting layer 220 may further include at least one of a hole injection layer (HIL), a hole transporting layer (HTL), an electron blocking layer (EBL) and an electron injection layer (EIL). Moreover, two or more light emitting layers may be disposed to be spaced apart from each other so that the OLED D may have a tandem structure.

The second electrode 230 is formed over the substrate 110 where the light emitting layer 220 is formed. The second electrode 230 covers an entire surface of the display area and may be formed of a conductive material having a relatively low work function to serve as a cathode. For example, the second electrode 230 may be formed of aluminum (Al), magnesium (Mg), calcium (Ca), silver (Ag) or their alloy, e.g., Mg-Ag alloy (MgAg). In the top-emission type organic light emitting display device 100, the second electrode 230 may have a thin profile to be transparent (or semi-transparent).

Although not shown, the organic light emitting display device 100 may further include a color filter. In the bottom-emission type organic light emitting display device 100, the color filter may be positioned between the OLED D and the substrate 110, e.g., between the interlayer insulating layer 132 and the planarization layer 150. Alternatively, in the top-emission type organic light emitting display device 100, the color filter may be positioned on or over the second electrode 230 of the OLED D.

An encapsulation film (or an encapsulation layer) 170 is formed on the second electrode 230 to prevent penetration of moisture into the OLED D. The encapsulation film 170 includes a first inorganic insulating layer 172, an organic insulating layer 174 and a second inorganic insulating layer 176 sequentially stacked, but it is not limited thereto.

The organic light emitting display device 100 may further include a polarization plate (not shown) for reducing an ambient light reflection. For example, the polarization plate may be a circular polarization plate. In the bottom-emission type organic light emitting display device 100, the polarization plate may be disposed under the substrate 110. In the top-emission type organic light emitting display device 100, the polarization plate may be disposed on or over the encapsulation film 170.

In addition, in the top-emission type organic light emitting display device 100, a cover window (not shown) may be attached to the encapsulation film 170 or the polarization plate. In this instance, the substrate 110 and the cover window have a flexible property such that a flexible organic light emitting display device may be provided.

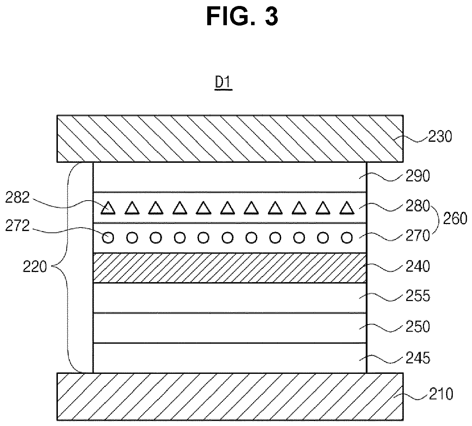

FIG. 3 is a schematic cross-sectional view of an OLED according to a second embodiment of the present disclosure.

As shown in FIG. 3, an OLED D1 includes a first electrode 210, a second electrode 230 facing the first electrode 210 and a light emitting layer 220 therebetween. The light emitting layer 220 includes an EML 240 and an intermediate organic layer 260 between the EML 240 and the second electrode 230. The intermediate organic layer 260 includes a first organic layer 270 and a second organic layer 280. The organic light emitting display device 100 (of FIG. 2) includes a red pixel region, a green pixel region and a blue pixel region, and the OLED D1 is positioned in each of the red, green and blue pixel regions.

The first electrode 210 may be an anode, and the second electrode 230 may be a cathode. One of the first and second electrodes 210 and 230 is a transparent electrode (semitransparent electrode), and the other one of the first and second electrodes 210 and 230 is a reflective electrode.

The light emitting layer 220 may further include at least one of an HTL 250 between the first electrode 210 and the EML 240 and an EBL 255 between the HTL 250 and the EML 240.

The light emitting layer 220 may further include at least one of an HIL 245 between the first electrode 210 and the HTL 250 and an EIL 290 between the second organic layer 280 of the intermediate organic layer 260 and the second electrode 230.

The HTL 250 may include at least one of the compounds selected from the group consisting ofN,N' -diphenyl-N,N' -bis(3-methylphenyl)-1,1' -biphenyl-4,4' -diamine (TPD), N,N' - diphenyl-N,N' -bis(1-naphthyl)-1,1' -biphenyl-4,4" -diamine (NPB or NPD), 4,4' -bis(N-carbazolyl)-1,1' -biphenyl (CBP), poly[N,N' -bis(4-butylphenyl)-N,N' -bis(phenyl)-benzidine] (Poly-TPD), poly[(9,9-dioctylfluorenyl-2,7-diyl)-co-(4,4' -(N-(4-sec-butylphenyl)diphenylamine))] (TFB), di-[4-(N,N-di-p-tolyl-amino)-phenyl]cyclohexane (TAPC), 3,5-di(9H-carbazol-9-yl)-N,N-diphenylaniline (DCDPA), N-(biphenyl-4-yl)-9,9-dimethyl-N-(4-(9-phenyl-9H-carbazol-3-yl)phenyl)-9H-fluoren-2-amine and N-(biphenyl-4-yl)-N-(4-(9-phenyl-9H-carbazol-3-yl)phenyl)biphenyl-4-amine, but it is not limited thereto. For example, the HTL 250 may have a thickness of 500 to 1500Å.

The HIL 245 may include at least one of the compounds selected from the group consisting of 4,4' ,4" -tris(3-methylphenylamino)triphenylamine (MTDATA), 4,4' ,4" -tris(N,N-diphenyl-amino)triphenylamine (NATA), 4,4' ,4" -tris(N-(naphthalene-1-yl)-N-phenyl-amino)triphenylamine (1T-NATA), 4,4' ,4" -tris(N-(naphthalene-2-yl)-N-phenyl-amino)triphenylamine (2T-NATA), copper phthalocyanine (CuPc), tris(4-carbazoyl-9-yl-phenyl)amine (TCTA), NPB (or NPD), 1,4,5,8,9,11-hexaazatriphenylenehexacarbonitrile(dipyrazino[2,3-f:2' 3' -h]quinoxaline-2,3,6,7,10,11-hexacarbonitrile (HAT-CN), 1,3,5-tris[4-(diphenylamino)phenyl]benzene (TDAPB), poly(3,4-ethylenedioxythiphene)polystyrene sulfonate (PEDOT/PSS) and N-(biphenyl-4-yl)-9,9-dimethyl-N-(4-(9-phenyl-9H-carbazol-3-yl)phenyl)-9H-fluoren-2-amine, but it is not limited thereto. Alternatively, the HIL 245 may include a material of the HTL 250 and a p-type dopant such as HAT-CN in Formula 4-1. The HIL 245 may have a thickness of 50 to 150Å.

The EBL 255 may include at least one of compounds selected from the group consisting of TCTA, tris[4-(diethylamino)phenyl]amine, N-(biphenyl-4-yl)-9,9-dimethyl-N-(4-(9-phenyl-9H-carbazol-3-yl)phenyl)-9H-fluoren-2-amine, TAPC, MTDATA, 1,3-bis(carbazol-9-yl)benzene (mCP), 3,3' -bis(N-carbazolyl)-1,1' -biphenyl (mCBP), CuPc, N,N' -bis[4-[bis(3-methylphenyl)amino]phenyl]-N,N' -diphenyl-[1,1' -biphenyl]-4,4' -diamine(DNTPD), TDAPB, DCDPA, 2,8-bis(9-phenyl-9H-carbazol-3-yl)dibenzo[b,d]thiophene and a compound in Formula 4-3, but it is not limited thereto. The EBL 255 may have a thickness of 100 to 200Å.

The EIL 290 may include Yb, LiF, CsF, NaF, BaF₂, or their alloy. The EIL 290 may have a thickness of 1 to 20Å.

The OLED D1 may further include a capping layer (not shown) under the first electrode 210 or on the second electrode 230. The capping layer may include an anthracene derivative (compound) and may have a thickness of 500 to 800Å. For example, the anthracene derivative of the capping layer may be a compound in Formula 4-6.

The EML 240 includes a host and a dopant (emitter). The EML 240 in the red pixel region includes a first host and a red dopant, the EML 240 in the green pixel region includes a second host and a green dopant, and the EML 240 in the blue pixel region includes a third host and a blue dopant. Each of the red, green and blue dopants may be at least one of a phosphorescent compound, a fluorescent compound and a delayed fluorescent compound. The EML 240 may have a thickness of 200 to 300Å.

Each of the first and second hosts may be independently selected from the group consisting of 9,9' -diphenyl-9H,9' H-3,3' -bicarbazole (BCzPh), CBP, 1,3,5-tris(carbazole-9-yl)benzene (TCP), TCTA, 4,4' -bis(carbazole-9-yl)-2,2' -dimethylbiphenyl (CDBP), 2,7-bis(carbazole-9-yl)-9,9-dimethylfluorene (DMFL-CBP), 2,2' ,7,7' -tetrakis(carbazole-9-yl)-9,9-spiorofluorene (Spiro-CBP), DPEPO, 4' -(9H-carbazol-9-yl)biphenyl-3,5-dicarbonitrile (PCzB-2CN), 3'-(9H-carbazol-9-yl)biphenyl-3,5-dicarbonitrile (mCzB-2CN), 3,6-bis(carbazole-9-yl)-9-(2-ethyl-hexyl)-9H-carbazole (TCz1), bis(2-hydroxylphenyl)-pyridine)beryllium (Bepp₂), bis(10-hydroxylbenzo[h] quinolinato)beryllium (Bebq₂) and 1,3,5-tris(1-pyrenyl)benzene (TPB₃).

The red dopant may be selected from the group consisting of [bis(2-(4,6-dimethyl)phenylquinoline)](2,2,6,6-tetramethylheptane-3,5-dionate)iridium(III), bis[2-(4-n-hexylphenyl)quinoline](acetylacetonate)iridium(III) (Hex-Ir(phq)₂(acac)), tris[2-(4-n-hexylphenyl)quinoline]iridium(III) (Hex-Ir(phq)₃), tris[2-phenyl-4-methylquinoline]iridium(III) (Ir(Mphq)₃), bis(2-phenylquinoline)(2,2,6,6-tetramethylheptene-3,5-dionate)iridium(III) (Ir(dpm)PQ₂), bis(phenylisoquinoline)(2,2,6,6-tetramethylheptene-3,5-dionate)iridium(III) (Ir(dpm)(piq)₂), bis[(4-n-hexylphenyl)isoquinoline](acetylacetonate)iridium(III) (Hex-Ir(piq)₂(acac)), tris[2-(4-n-hexylphenyl)quinoline]iridium(III) (Hex-Ir(piq)₃), tris(2-(3-methylphenyl)-7-methyl-quinolato)iridium (Ir(dmpq)₃), bis[2-(2-methylphenyl)-7-methylquinoline](acetylacetonate)iridium(III) (Ir(dmpq)₂(acac)) and bis[2-(3,5-dimethylphenyl)-4-methyl-quinoline](acetylacetonate)iridium(III) (Ir(mphmq)₂(acac)).

The green dopant may be selected from the group consisting of [Bis(2-phenylpyridine)](pyridyl-2-benzofuro[2,3-b]pyridine)iridium, fac-tris(2-phenylpyridine)iridium( III) (fac-Ir(ppy)₃), bis(2-phenylpyridine)(acetylacetonate)iridium(III) (Ir(ppy)₂(acac)), tris[2-(p-tolyl)pyridine]iridium(III) (Ir(mppy)₃), bis(2-(naphthalene-2-yl)pyridine)(acetylacetonate)iridium(III) (Ir(npy)₂acac), tris(2-phenyl-3-methyl-pyridine)iridium (Ir(3mppy)₃) and fac-tris(2-(3-p-xylyl)phenyl)pyridine iridium(III) (TEG).

The third host may be selected from the group consisting of mCP, 9-(3-(9H-carbazol-9-yl)phenyl)-9H-carbazole-3-carbonitrile (mCP-CN), mCBP, CBP-CN, 9-(3-(9H-carbazol-9-yl)phenyl)-3-(diphenylphosphoryl)-9H-carbazole (mCPPO1), 3,5-di(9H-carbazol-9-yl)biphenyl (Ph-mCP), TSPO1, 9-(3'-(9H-carbazol-9-yl)-[1,1'-biphenyl]-3-yl)-9H-pyrido[2,3-b]indole (CzBPCb), bis(2-methylphenyl)diphenylsilane (UGH-1), 1,4-bis(triphenylsilyl)benzene (UGH-2), 1,3-bis(triphenylsilyl)benzene (UGH-3), 9,9-spiorobifluoren-2-yl-diphenyl-phosphine oxide (SPPO1), 9,9'-(5-(triphenylsilyl)-1,3-phenylene)bis(9H-carbazole) (SimCP) and an anthracene derivative, e.g., a compound in Formula 4-4. In addition, the blue dopant may be selected from the group consisting of perylene, 4,4'-bis[4-(di-p-tolylamino)styryl]biphenyl (DPAVBi), 4-(di-p-tolylamino)-4-4'-[(di-p-tolylamino)styryl]stilbene (DPAVB), 4,4'-bis[4-(diphenylamino)styryl]biphenyl (BDAVBi), 2,7-bis(4-diphenylamino)styryl-9,9-spiorfluorene (spiro-DPVBi), [1,4-bis[2-[4-[N,N-di(p-tolyl)amino]phenyl]vinyl] benzene (DSB), 1-4-di-[4-(N,N-diphenyl)amino]styryl-benzene (DSA), 2,5,8,11-tetra-tetr-butylperylene (TBPe), bis(2-hydroxylphenyl-pyridine)beryllium (Bepp₂), 9-(9-phenylcarbazole-3-yl)-10-(naphthalene-1-yl)anthracene (PCAN), mer-tris(1-phenyl-3-methylimidazolin-2-ylidene-C,C(2)'iridium(III) (mer-Ir(pmi)₃), fac-tris(1,3-diphenyl-benzimidazolin-2-ylidene-C,C(2)'iridium(III) (fac-Ir(dpbic)₃), bis(3,4,5-trifluoro-2-(2-pyridyl)phenyl-(2-carboxypyridyl)iridium(III) (Ir(tfpd)₂pic), tris(2-(4,6-difluorophenyl)pyridine)iridium(III) (Ir(Fppy)₃), bis[2-(4,6-difluorophenyl)pyridinato-C2,N](picolinato)iridium(III) (FIrpic) and a pyrene derivative, e.g., a compound in Formula 4-5.

In the intermediate organic layer 260, the first organic layer 270 is positioned between the EML 240 and the EIL 290 or between the EML 240 and the second electrode 230, and the second organic layer 280 is positioned between the first organic layer 270 and the EIL 290 or between the first organic layer 270 and the second electrode 230. The first and second organic layers 270 and 280 contact each other.

For example, the first organic layer 270 may be an HBL, and the second organic layer 280 may be an ETL.

The intermediate organic layer 260 includes a first compound 272 having a structure in which a pyrimidine moiety and a quinoline moiety directly linked (connected, combined or joined) to each other. For example, the first organic layer 270 includes the first compound 272. The first compound 272 is represented by Formula 1-1.

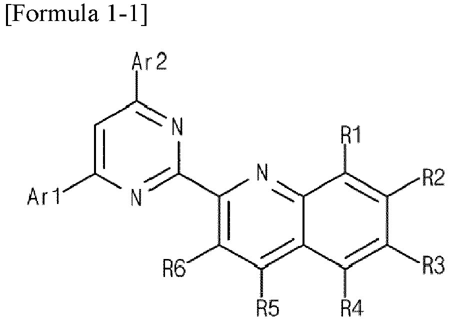

In Formula 1-1, each of Ar1 and Ar2 is independently selected from the group consisting of a substituted or unsubstituted C6 to C50 aryl group and a substituted or unsubstituted C5 to C50 heteroaryl group. Each of R1 to R6 is independently selected form the group consisting of hydrogen, deuterium, a substituted or unsubstituted C1 to C10 alkyl group, a substituted or unsubstituted C6 to C30 aryl group and a substituted or unsubstituted heteroaryl group.

In the present disclosure, without specific definition, the C6 to C50 aryl group and/or C6 to C30 aryl group may be selected from the group consisting of phenyl, biphenyl, terphenyl, naphthyl, anthracenyl, pentanenyl, indenyl, indenoindenyl, heptalenyl, biphenylenyl, indacenyl, phenanthrenyl, benzophenanthrenyl, dibenzophenanthrenyl, azulenyl, pyrenyl, fluoranthenyl, triphenylenyl, chrysenyl, tetraphenyl, tetracenyl, picenyl, pentaphenyl, pentacenyl, fluorenyl, indenofluorenyl and spiro-fluorenyl.

In the present disclosure, without specific definition, the C5 to C50 heteroaryl group and/or the C5 to C30 heteroaryl group may be selected from the group consisting of pyrrolyl, pyridinyl, pyrimidinyl, pyrazinyl, pyridazinyl, triazinyl, tetrazinyl, imidazolyl, pyrazolyl, indolyl, isoindolyl, indazolyl, indolizinyl, pyrrolizinyl, carbazolyl, benzocarbazolyl, dibenzocarbazolyl, indolocarbazolyl, indenocarbazolyl, benzofurocarbazolyl, benzothienocarbazolyl, quinolinyl, isoquinolinyl, phthalazinyl, quinoxalinyl, cinnolinyl, quinazolinyl, quinozolinyl, purinyl, benzoquinolinyl, benzoisoquinolinyl, benzoquinazolinyl, benzoquinoxalinyl, acridinyl, phenanthrolinyl, perimidinyl, phenanthridinyl, pteridinyl, naphtharidinyl, furanyl, oxazinyl, oxazolyl, oxadiazolyl, triazolyl, dioxynyl, benzofuranyl, dibenzofuranyl, thiopyranyl, xanthenyl, chromanyl, isochromanyl, thioazinyl, thiophenyl, benzothiophenyl, dibenzothiophenyl, difuropyrazinyl, benzofurodibenzofuranyl, benzothienobenzothiophenyl, benzothienodibenzothiophenyl, benzothienobenzofuranyl, and benzothienodibenzofuranyl.

In the present disclosure, without specific definition, a substituent of an alkyl group, an aryl group and/or a heteroaryl group may be at least one of deuterium, tritium, halogen, cyano, a C1 to C20 alkyl group, aryl phosphine oxide, a substituted or unsubstituted C6 to C30 aryl group and a substituted or unsubstituted C5 to C30 heteroaryl group.

For example, in Formula 1-1, one of Ar1 and Ar2 may be a C6 to C50 aryl group, e.g., phenyl, unsubstituted or substituted with a C1 to C20 alkyl group, e.g., tert-butyl, and the other one of Ar1 and Ar2 may be C6 to C50 aryl group, e.g., phenyl, naphthyl, biphenyl, fluorenyl, unsubstituted or substituted with at least one of a substituted or unsubstituted C6 to C30 aryl group or a substituted or unsubstituted C5 to C30 heteroaryl group.

In one embodiment, each of Ar1 and Ar2 is independently selected form the group consisting of a substituted or unsubstituted C6 to C20 aryl group and a substituted or unsubstituted C5 to C20 heteroaryl group. Preferably, each of R1 to R6 is independently selected form the group consisting of hydrogen, deuterium, and a substituted or unsubstituted C1 to C10 alkyl group, more preferably each of R1 to R6 is hydrogen.

In one embodiment, at least one of Ar1 and Ar2 is phenyl and each of R1 to R6 is hydrogen.

The first compound 272 may be one of the compounds in Formula 1-2.

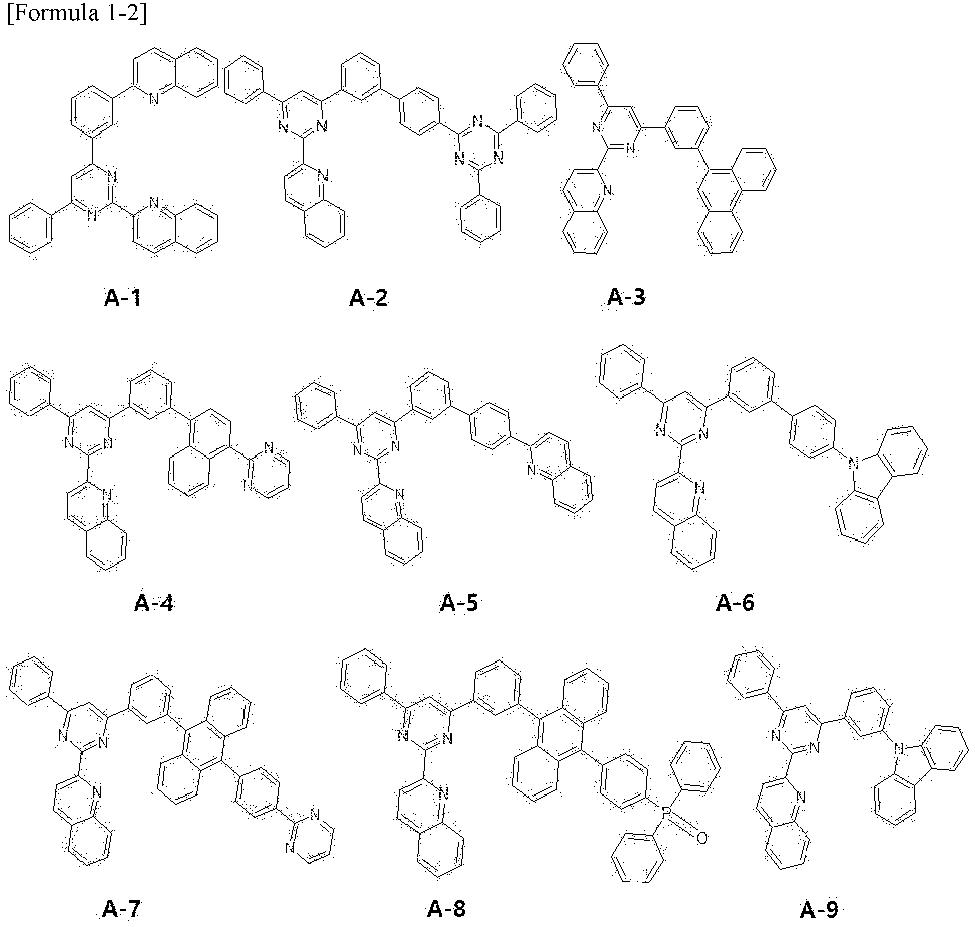

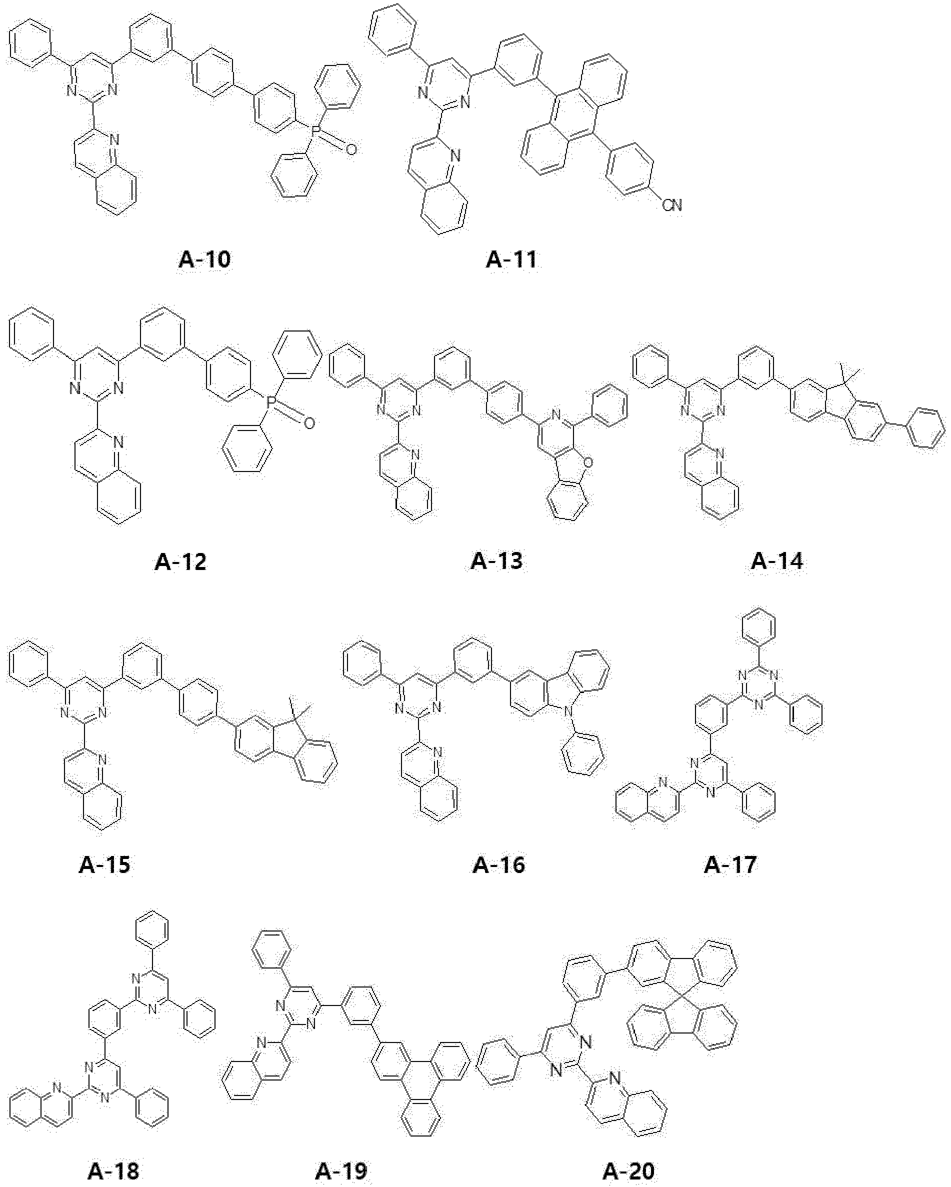

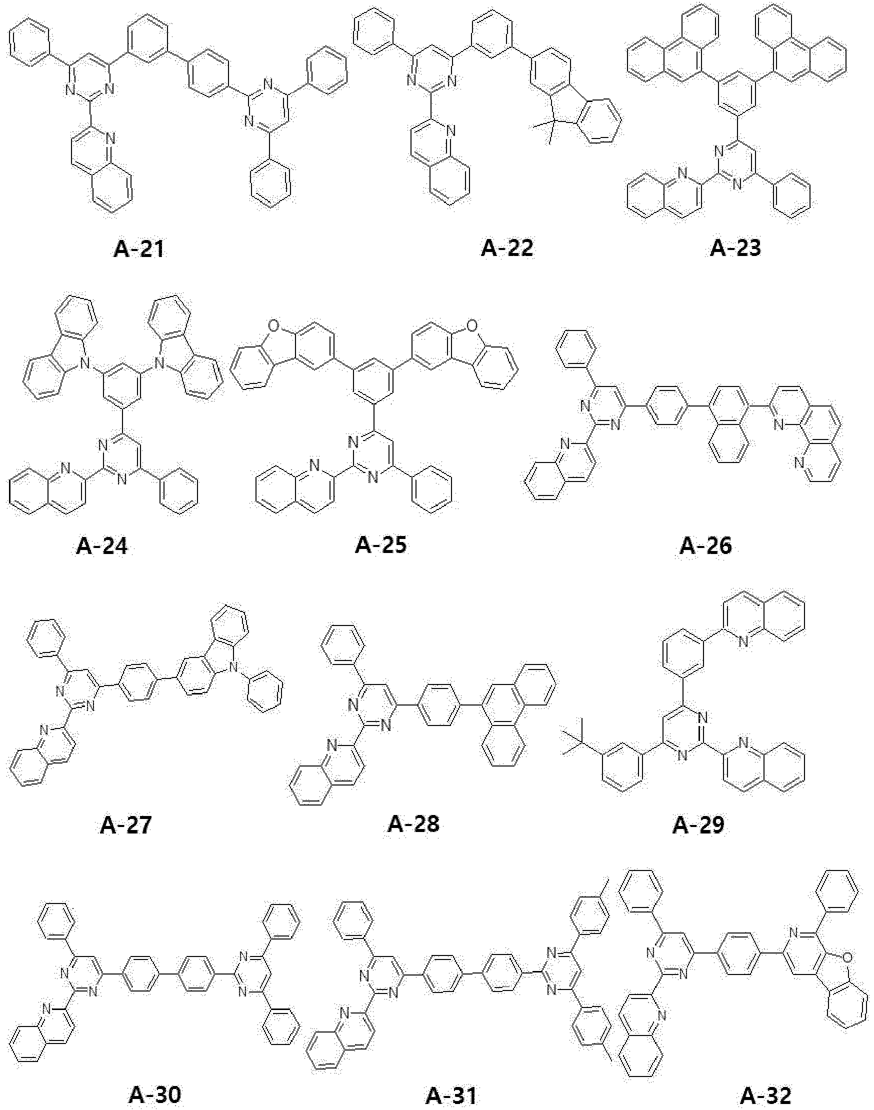

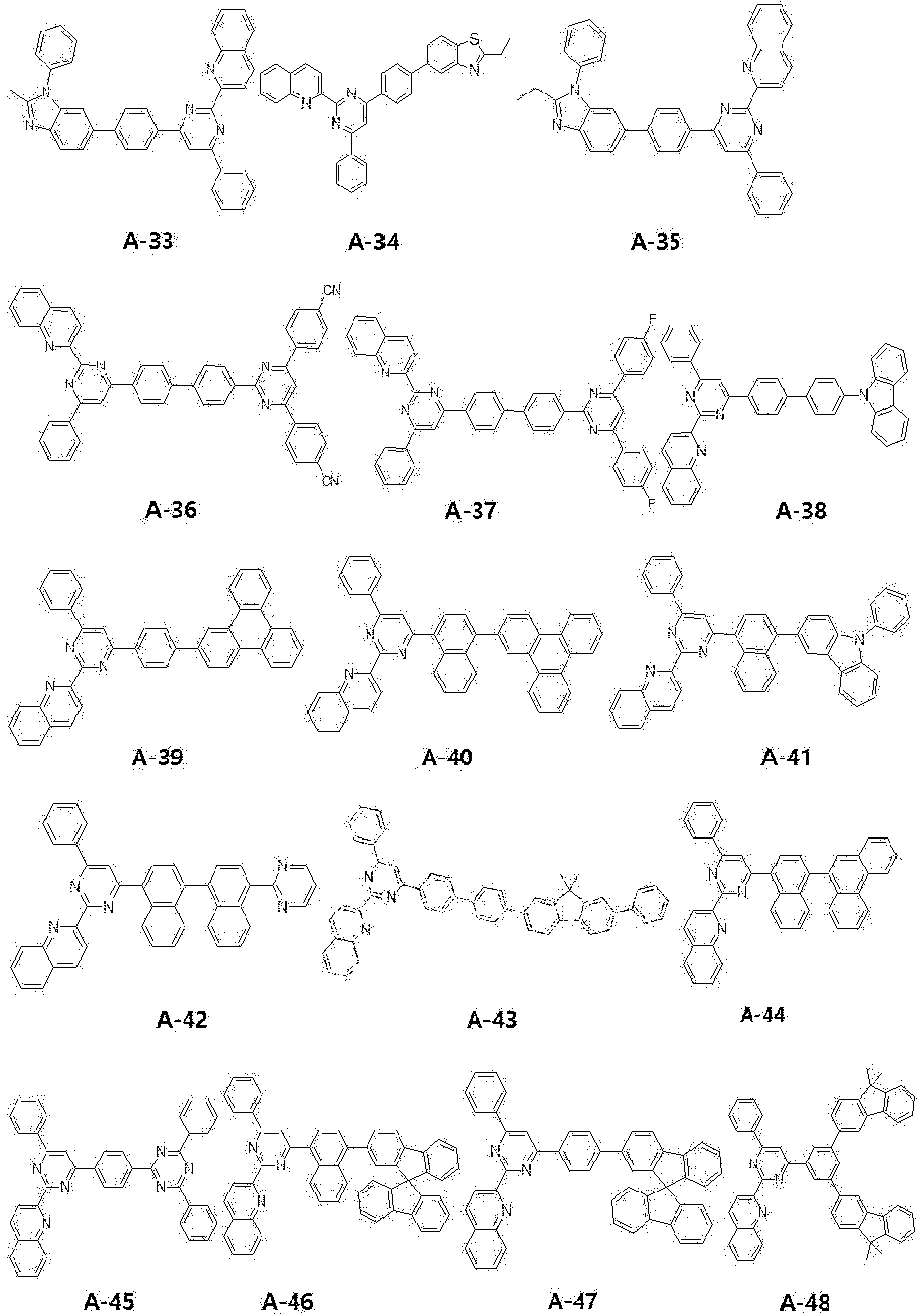

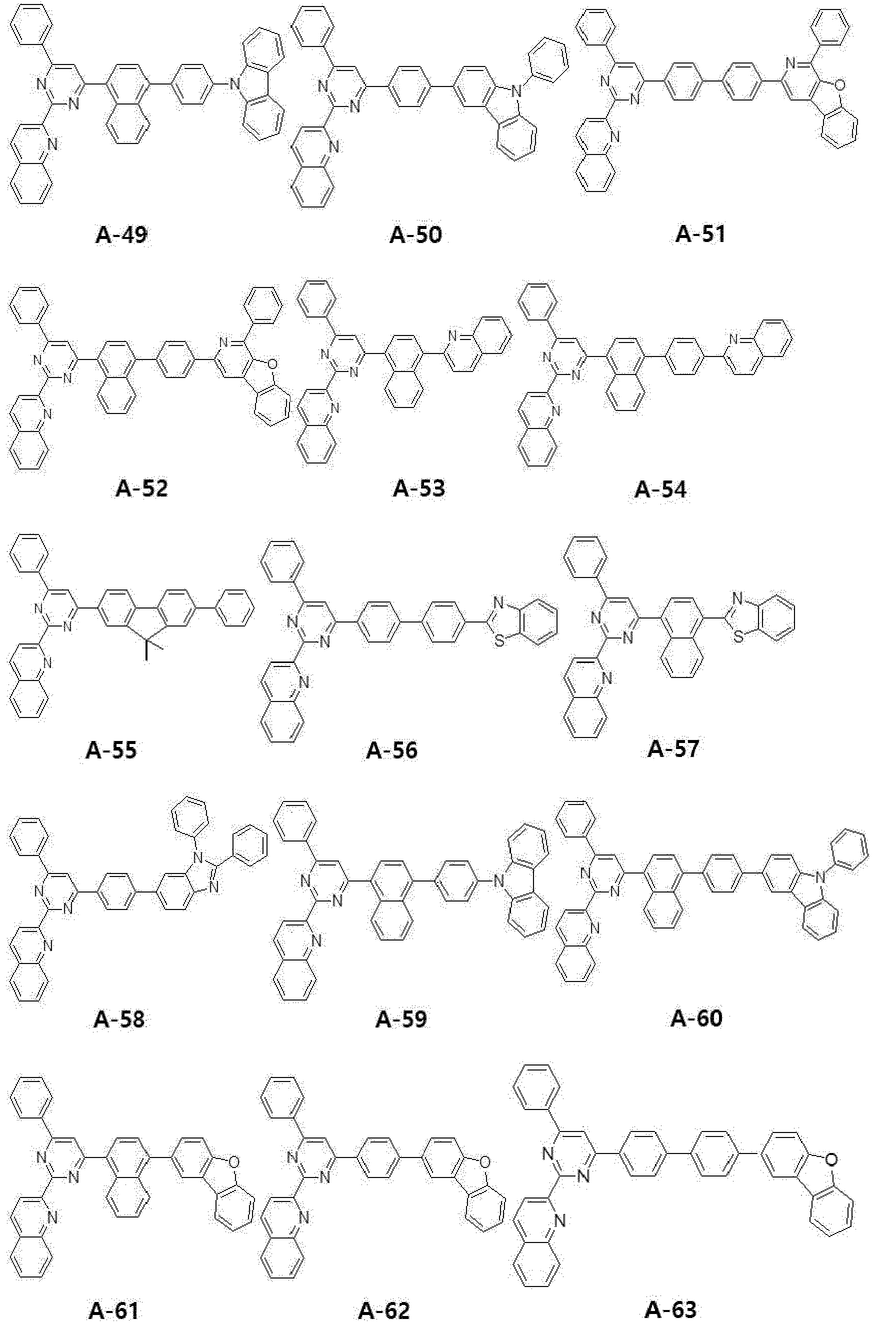

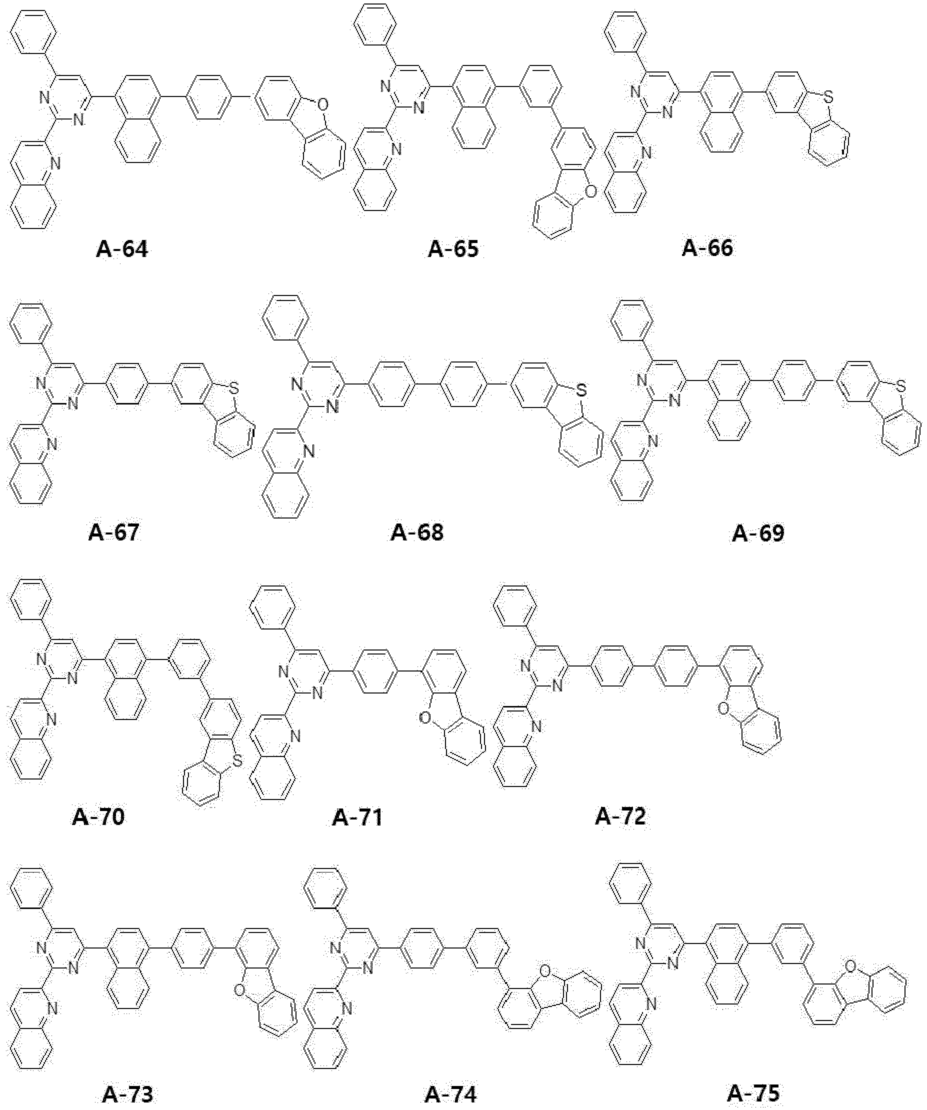

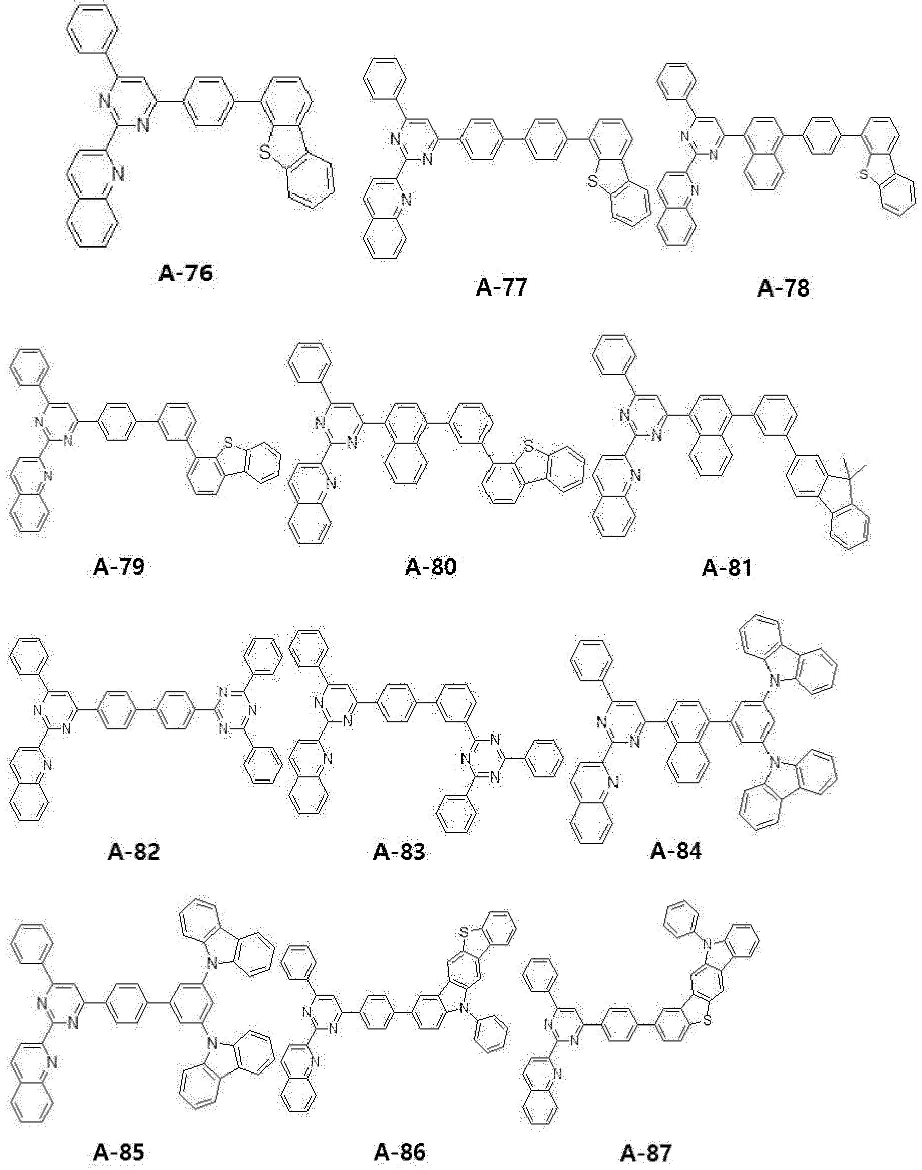

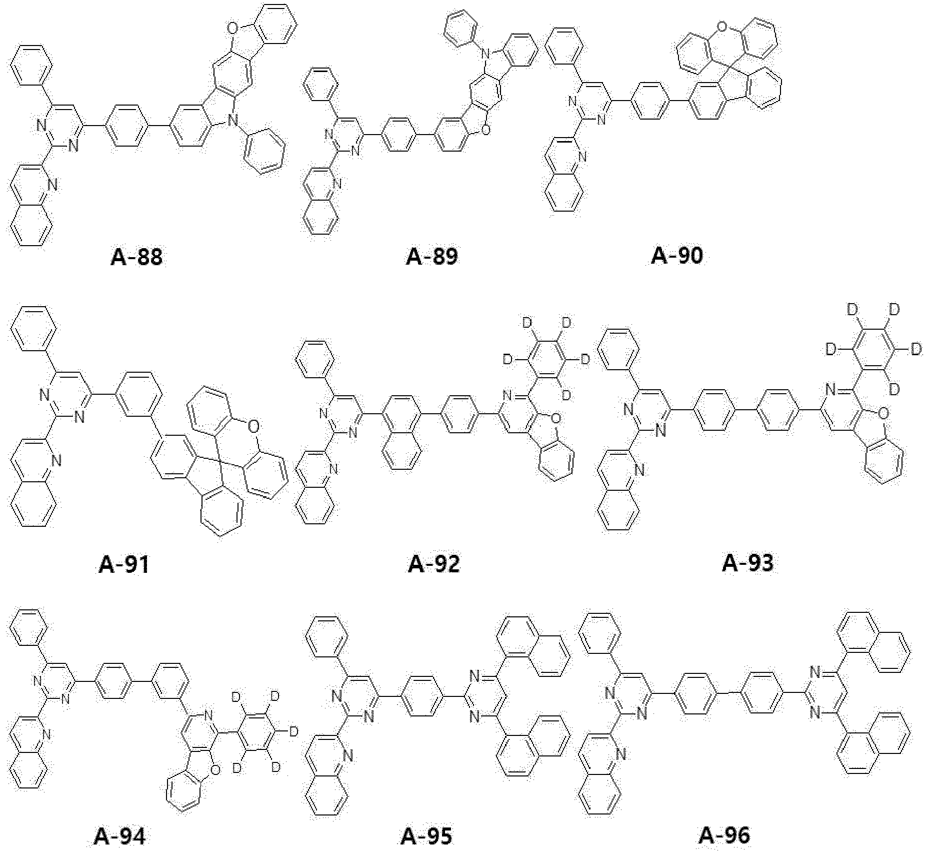

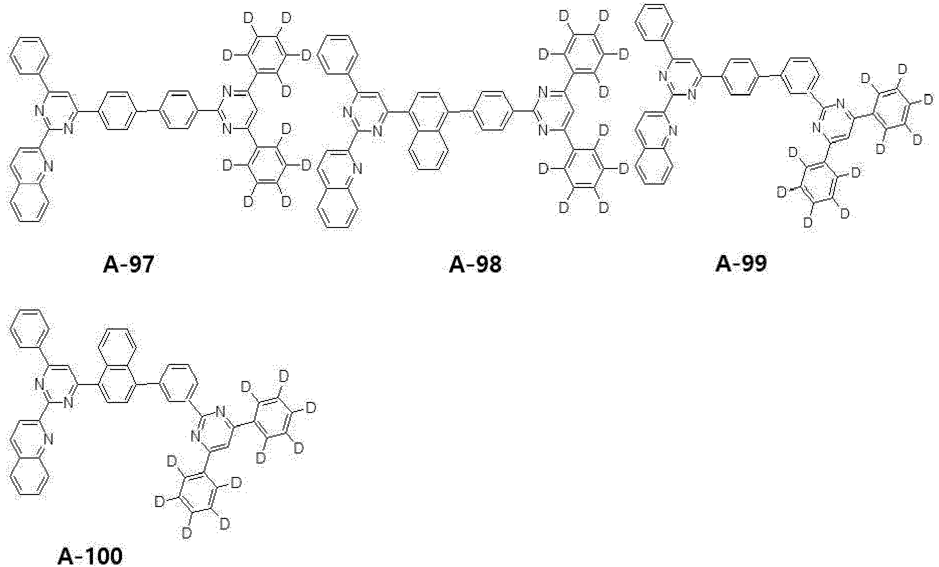

Preferred compounds are A-3, A-20 and A-83.

The intermediate organic layer 260 further includes a second compound 282 being a pyrimidine derivative, a triazine derivative or an anthracene derivative. For example, the second organic layer 280 may include the second compound 282. The second compound 282 is represented by one of Formula 2-1, Formula 2-2 and Formula 2-3.

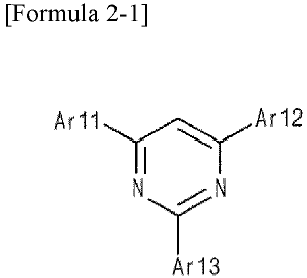

In Formula 2-1, two of Ar11 to Ar13 are independently selected from a substituted or unsubstituted C6 to C30 aryl group, and the other one of Ar11 to Ar13 is selected from the group consisting of a C6 to C30 aryl group substituted with a substituted or unsubstituted C5 to C30 heteroaryl group and a substituted or unsubstituted C5 to C30 heteroaryl group. The C6 to C30 aryl group and the C5 to C30 heteroaryl group may be selected from the groups listed above in view of [Formula 1-1].

In one embodiment, two of Ar11 to Ar13 are independently selected from an unsubstituted C6 to C30 aryl group, and the other one of Ar11 to Ar13 is selected from the group consisting of a C6 to C30 aryl group substituted with an unsubstituted C5 to C30 heteroaryl group and an unsubstituted C5 to C30 heteroaryl group.

In one embodiment, two of Ar11 to Ar13 are is independently selected from an unsubstituted C6 to C18 aryl group, and the other one of Ar11 to Ar13 is selected from the group consisting of a C6 to C30 aryl group substituted with an unsubstituted C5 to C30 heteroaryl group and an unsubstituted C5 to C30 heteroaryl group.

In one embodiment, two of Ar11 to Ar13 are independently selected from an unsubstituted C6 to C18 aryl group, and the other one of Ar11 to Ar13 is a phenyl substituted with an unsubstituted C5 to C30 heteroaryl group.

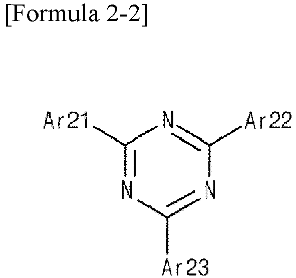

In Formula 2-2, each of Ar21 and Ar22 is independently selected from a substituted or unsubstituted C6 to C30 aryl group, and Ar23 is selected from the group consisting of a C6 to C30 aryl group unsubstituted or substituted with a C5 to C30 heteroaryl group and a substituted or unsubstituted C5 to C30 heteroaryl group. The C6 to C30 aryl group and the C5 to C30 heteroaryl group may be selected from the groups listed above in view of [Formula 1-1].

In one embodiment, each of Ar21 and Ar22 is independently selected from an unsubstituted C6 to C30 aryl group, and Ar23 is selected from the group consisting of a C6 to C30 aryl group substituted with an unsubstituted C5 to C30 heteroaryl group and an unsubstituted C5 to C30 heteroaryl group.

In one embodiment, each of Ar21 and Ar22 is independently selected from an unsubstituted C6 to C18 aryl group, and Ar23 is selected from the group consisting of a C6 to C30 aryl group substituted with an unsubstituted C5 to C30 heteroaryl group and an unsubstituted C5 to C30 heteroaryl group.

In one embodiment, each of Ar21 and Ar22 is independently selected from an unsubstituted C6 to C18 aryl group, and Ar23 is a phenyl substituted with an unsubstituted C5 to C30 heteroaryl group.

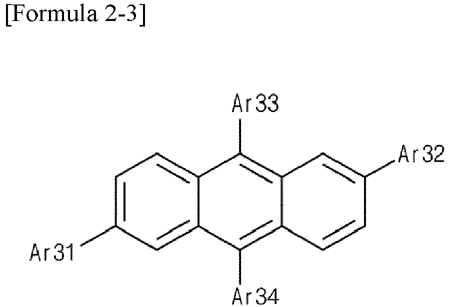

In Formula 2-3, each of Ar31 and Ar32 is independently selected from the group consisting of hydrogen, a substituted or unsubstituted C6 to C30 aryl group and a substituted or unsubstituted C5 to C30 heteroaryl group, and each of Ar33 and Ar34 is independently selected from the group consisting of a substituted or unsubstituted C6 to C30 aryl group and a substituted or unsubstituted C5 to C30 heteroaryl group. The C6 to C30 aryl group and the C5 to C30 heteroaryl group may be selected from the groups listed above in view of [Formula 1-1].

In one embodiment, each of Ar31 and Ar32 is independently selected from the group consisting of hydrogen, an unsubstituted C6 to C30 aryl group and an unsubstituted C5 to C30 heteroaryl group, and each of Ar33 and Ar34 is independently selected from the group consisting of a substituted or unsubstituted C6 to C20 aryl group and a substituted or unsubstituted C5 to C20 heteroaryl group.

In one embodiment, each of Ar31 and Ar32 is independently selected from the group consisting of hydrogen, an unsubstituted C6 to C20 aryl group and an unsubstituted C5 to C20 heteroaryl group, and each of Ar33 and Ar34 is independently selected from the group consisting of a substituted or unsubstituted C6 to C20 aryl group and a substituted or unsubstituted C5 to C20 heteroaryl group.

In one embodiment, at least one of Ar31 and Ar32 is hydrogen. In another embodiment, at least one of Ar31 and Ar32 is phenyl or an unsubstituted C5 to C20 heteroaryl group.

In one embodiment, Ar31 and Ar32 are identical, and also Ar33 and Ar34 are identical.

The second compound 282 may be one of the compounds in Formula 2-4.

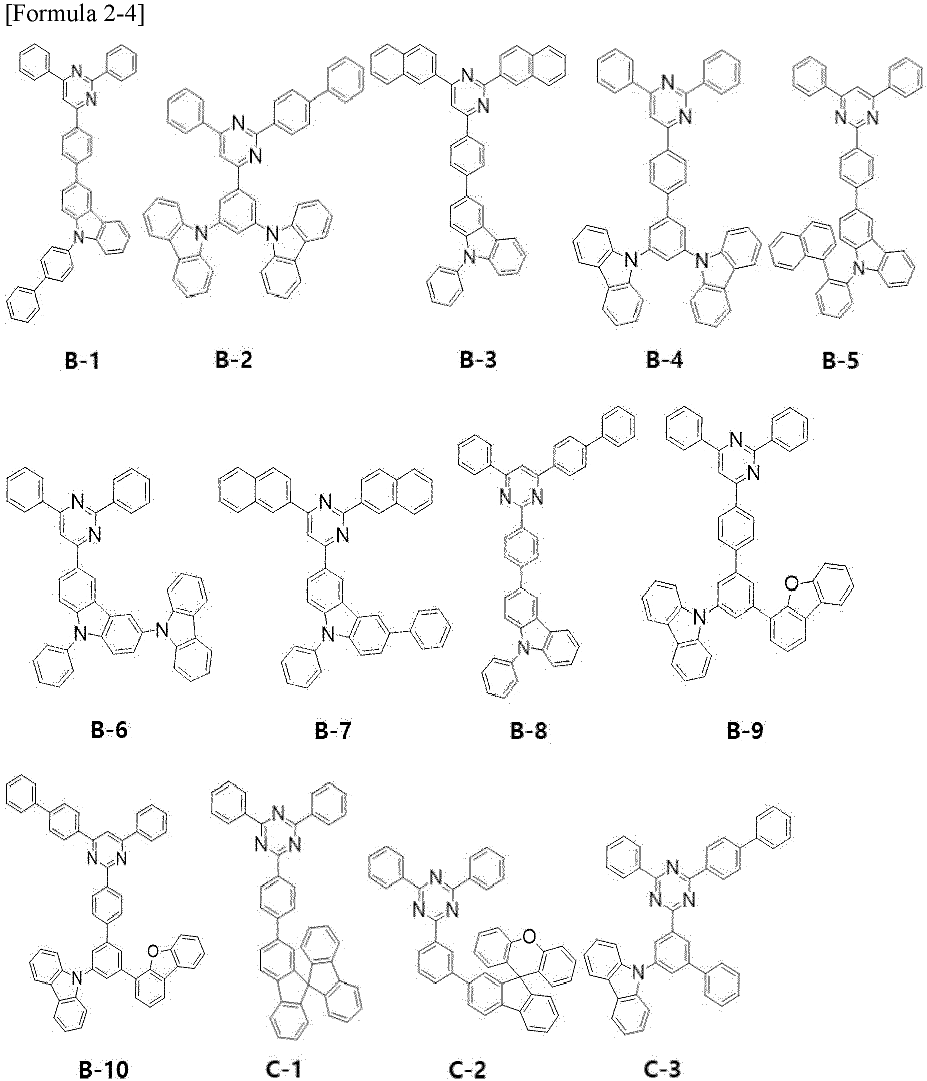

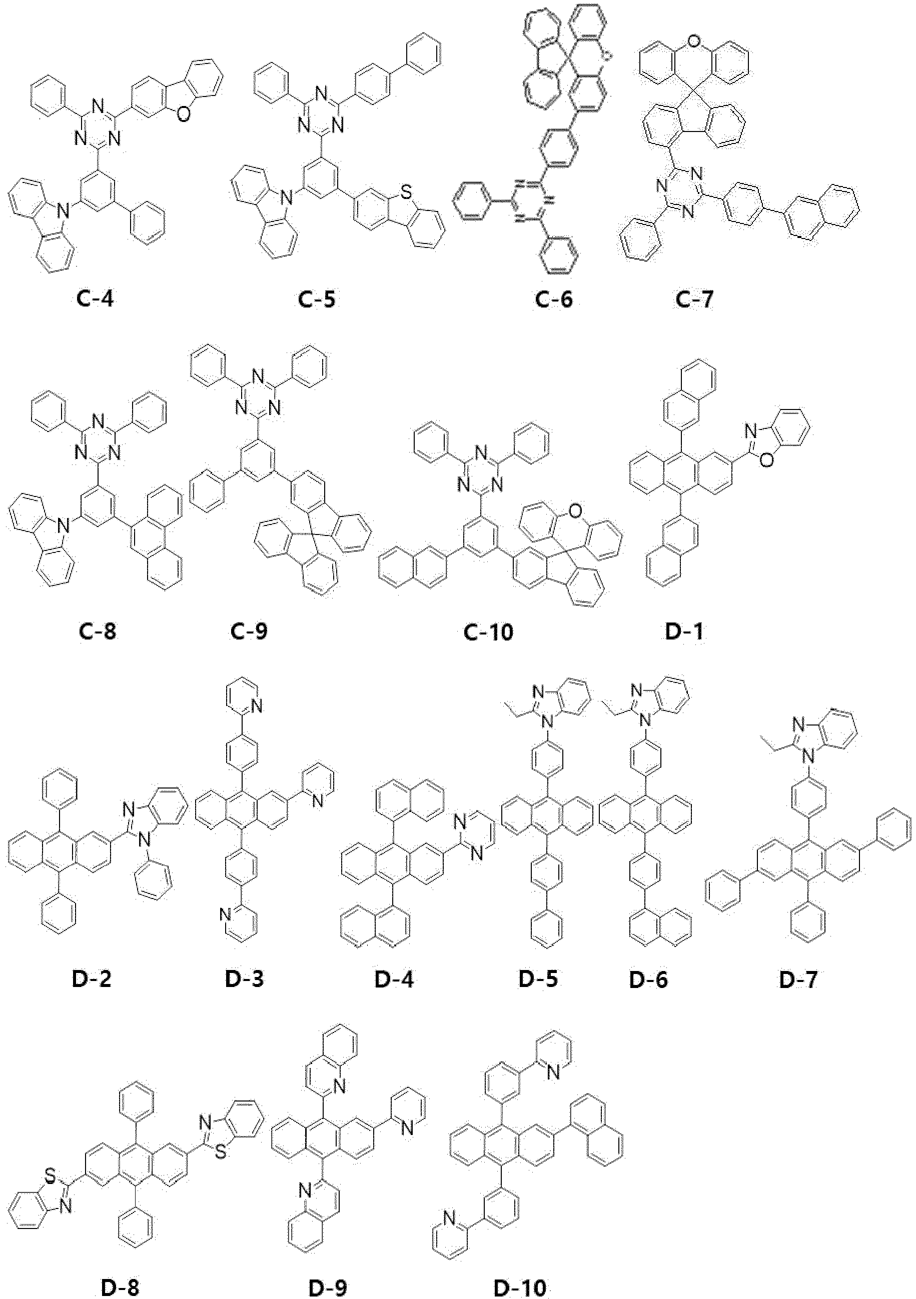

Preferred compounds are B-2, C-7 and D-5.

Each of the compounds of [Formula 2-1], [Formula 2-2] and [Formula 2-3] can be combined with any compound of [Formula 1-1].

The second organic layer 280 may further include a third compound (not shown) being a lithium (Li) derivative. For example, the Li derivative may be the compound, lithium quinolate (Liq), in Formula 3.

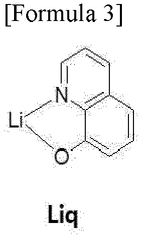

The third compound may have a volume % of 10 to 200, preferably 50 to 150, with respect to the second compound 282.

The first organic layer 270 has a first thickness, and the second organic layer 280 has a second thickness being greater than the first thickness. For example, the first thickness may be 30 to 100Å, and the second thickness may be 200 to 300Å.

As illustrated above, the OLED D1 of the present disclosure includes the intermediate organic layer 260, which includes the first and second organic layers 270 and 280, between the EML 240 and the second electrode 230 being the cathode, and the first organic layer 270 includes the first compound 272 represented by Formula 1-1. Accordingly, the turn-on voltage and the driving voltage of the OLED D1 are decreased so that the OLED D1 and the organic light emitting display device 100 including the OLED D1 have advantages of decrease of power consumption, increase of emitting efficiency and increase of lifespan.

In addition, since the second organic layer 280 includes the second compound 282 represented by one of Formulas 2-1 to 2-3, the OLED D1 and the organic light emitting display device 100 including the OLED D1 have big advantages of decrease of power consumption, increase of emitting efficiency and increase of lifespan.

Moreover, since the second organic layer 280 includes the third compound being the Li derivative, the property of the OLED D1 and the organic light emitting display device 100 including the OLED D1 is further improved.

### [OLED]

An anode (ITO), an HIL (the compound in Formula 4-1 (12vol%) and the compound in Formula 4-2, 100Å), a HTL (the compound in Formula 4-2, 930Å), a EBL (the compound in Formula 4-3, 150Å), a EML (the compound in Formula 4-4 (host) and the compound in Formula 4-5 (dopant, 3vol%), 250Å), a HBL (60Å), a ETL (250Å), an EIL (Yb:LiF, 15Å), a cathode (Ag:Mg, 145Å) and a capping layer (the compound in Formula 4-6, 650Å) are sequentially deposited to form an OLED.

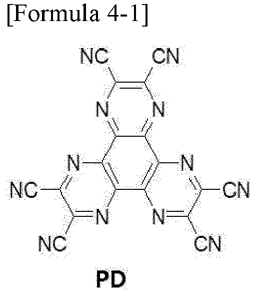

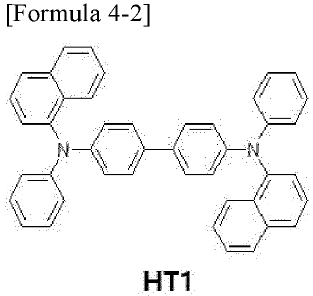

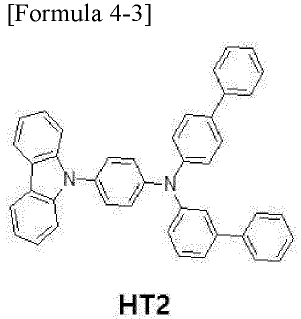

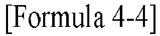

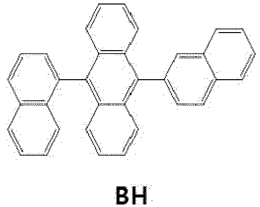

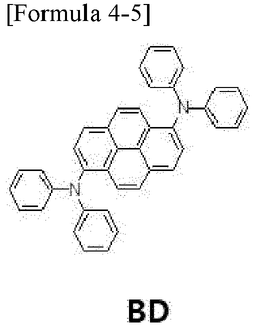

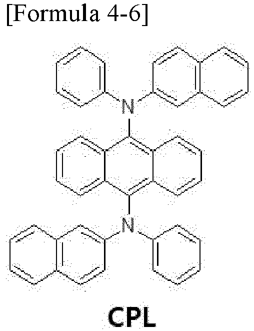

### 1. Comparative Example

### (1) Comparative Example 1 (Ref1)

The compound E-1 in Formula 5 is used to form the HBL, and the compound ET in Formula 6 and the compound (Liq) in Formula 3 are used to form the ETL. (a volume ratio (vol%), ET:Liq=1:1)

### (2) Comparative Example 2 (Ref2)

The compound E-2 in Formula 5 is used to form the HBL, and the compound ET in Formula 6 and the compound (Liq) in Formula 3 are used to form the ETL. (a volume ratio (vol%), ET:Liq=1:1)

### (3) Comparative Example 3 (Ref3)

The compound E-3 in Formula 5 is used to form the HBL, and the compound ET in Formula 6 and the compound (Liq) in Formula 3 are used to form the ETL. (a volume ratio (vol%), ET:Liq=1:1)

### 2. Example 1 (Ex1)

The compound A-3 in Formula 1-2 is used to form the HBL, and the compound ET in Formula 6 and the compound (Liq) in Formula 3 are used to form the ETL. (a volume ratio (vol%), ET:Liq=1:1)

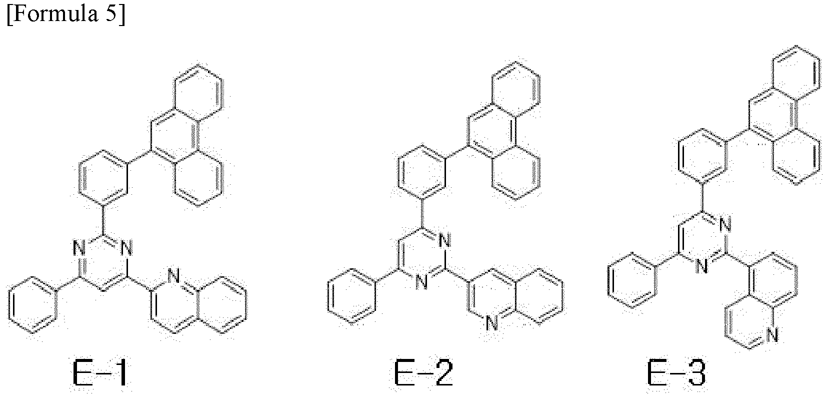

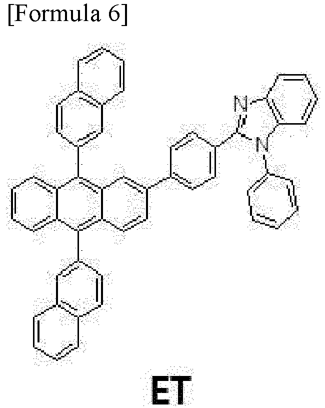

The emitting properties, i.e., the driving voltage (V), the quantum efficiency (QE), the blue index (cd/A/CIEy) and the lifespan (T95), of the OLED in Comparative Examples 1 to 3 and Example 1 are measured and listed in Table 1.

**Table 1**

| | HBL | ETL (Liq vol ratio) | V | QE | cd/A/CIEy | T95 |
|---|---|---|---|---|---|---|
| Ref1 | E-1 | ET (1:1) | 107% | 91% | 89% | 90% |
| Ref2 | E-2 | ET (1:1) | 105% | 99% | 97% | 95% |
| Ref3 | E-3 | ET (1:1) | 106% | 99% | 97% | 97% |
| Ex1 | A-3 | ET (1:1) | 100% | 100% | 100% | 100% |

The first compound in Formula 1-1 of the present disclosure has a structure in which a 2^{nd}-position of a pyrimidine moiety and a 2^{nd}-position of a quinoline moiety directly linked to each other, while the compounds E-1, E-2 and E-3 used in the first organic layer, i.e., the HBL, of the OLED of Ref1 to Ref3 have different structure from the first compound of the present disclosure.

As shown in Table 1, in comparison to the OLED of Ref1 to Ref3, in the OLED of Ex1, in which the first organic layer includes the first compound in Formula 1-1, the driving voltage is reduced, and the quantum efficiency, the blue index and the lifespan are improved.

### 3. Comparative Example 4 (Ref4)

The compound HB in Formula 7 is used to form the HBL, and the compound ET in Formula 6 and the compound (Liq) in Formula 3 are used to form the ETL. (a volume ratio (vol%), ET:Liq=1:1)

### 4. Example

### (1) Example 2 (Ex2)

The compound A-3 in Formula 1-2 is used to form the HBL, and the compound ET in Formula 6 and the compound (Liq) in Formula 3 are used to form the ETL. (a volume ratio (vol%), ET:Liq=1:1)

### (2) Example 3 (Ex3)

The compound A-3 in Formula 1-2 is used to form the HBL, and the compound D-5 in Formula 2-4 and the compound (Liq) in Formula 3 are used to form the ETL. (a volume ratio (vol%), D-5:Liq=2:1)

### (3) Example 4 (Ex4)

The compound A-20 in Formula 1-2 is used to form the HBL, and the compound D-5 in Formula 2-4 and the compound (Liq) in Formula 3 are used to form the ETL. (a volume ratio (vol%), D-5:Liq=2:1)

### (4) Example 5 (Ex5)

The compound A-83 in Formula 1-2 is used to form the HBL, and the compound D-5 in Formula 2-4 and the compound (Liq) in Formula 3 are used to form the ETL. (a volume ratio (vol%), D-5:Liq=2:1)

### (5) Example 6 (Ex6)

The compound A-83 in Formula 1-2 is used to form the HBL, and the compound D-5 in Formula 2-4 and the compound (Liq) in Formula 3 are used to form the ETL. (a volume ratio (vol%), D-5:Liq=1:1)

### (6) Example 7 (Ex7)

The compound A-3 in Formula 1-2 is used to form the HBL, and the compound B-2 in Formula 2-4 and the compound (Liq) in Formula 3 are used to form the ETL. (a volume ratio (vol%), B-2:Liq=1:1)

### (7) Example 8 (Ex8)

The compound A-20 in Formula 1-2 is used to form the HBL, and the compound B-2 in Formula 2-4 and the compound (Liq) in Formula 3 are used to form the ETL. (a volume ratio (vol%), B-2:Liq=1:1)

### (8) Example 9 (Ex9)

The compound A-20 in Formula 1-2 is used to form the HBL, and the compound B-2 in Formula 2-4 and the compound (Liq) in Formula 3 are used to form the ETL. (a volume ratio (vol%), B-2:Liq=1:2)

### (9) Example 10 (Ex10)

The compound A-83 in Formula 1-2 is used to form the HBL, and the compound B-2 in Formula 2-4 and the compound (Liq) in Formula 3 are used to form the ETL. (a volume ratio (vol%), B-2:Liq=1:1)

### (10) Example 11 (Ex11)

The compound A-3 in Formula 1-2 is used to form the HBL, and the compound C-7 in Formula 2-4 and the compound (Liq) in Formula 3 are used to form the ETL. (a volume ratio (vol%), C-7:Liq=1:2)

### (11) Example 12 (Ex12)

The compound A-3 in Formula 1-2 is used to form the HBL, and the compound C-7 in Formula 2-4 and the compound (Liq) in Formula 3 are used to form the ETL. (a volume ratio (vol%), C-7:Liq=1:1)

### (12) Example 13 (Ex13)

The compound A-20 in Formula 1-2 is used to form the HBL, and the compound C-7 in Formula 2-4 and the compound (Liq) in Formula 3 are used to form the ETL. (a volume ratio (vol%), C-7:Liq=1:2)

### (13) Example 14 (Ex14)

The compound A-83 in Formula 1-2 is used to form the HBL, and the compound C-7 in Formula 2-4 and the compound (Liq) in Formula 3 are used to form the ETL. (a volume ratio (vol%), C-7:Liq=1:2)

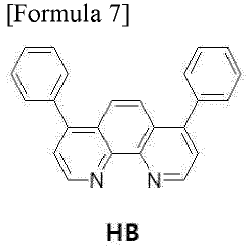

The emitting properties, i.e., the driving voltage (V), the luminance (cd/A), the quantum efficiency (QE), the blue index (cd/A/CIEy) and the lifespan (T95), of the OLED in Comparative Example 4 and Examples 2 to 14 are measured and listed in Table 2.

**Table 2**

| | HBL | ETL (Liq vol ratio) | V | cd/A | QE | cd/A/CIEy | T95 |
|---|---|---|---|---|---|---|---|
| Ref4 | HB | ET (1:1) | 100% | 100% | 100% | 100% | 100% |
| Ex2 | A-3 | ET (1:1) | 97% | 104% | 106% | 106% | 102% |
| Ex3 | A-3 | D-5 (2:1) | 97% | 108% | 110% | 113% | 105% |
| Ex4 | A-20 | D-5 (2:1) | 86% | 116% | 118% | 119% | 102% |
| Ex5 | A-83 | D-5 (2:1) | 86% | 117% | 118% | 119% | 100% |
| Ex6 | A-83 | D-5 (1:1) | 88% | 118% | 119% | 120% | 101% |
| Ex7 | A-3 | B-2 (1:1) | 90% | 104% | 107% | 109% | 114% |
| Ex8 | A-20 | B-2 (1:1) | 94% | 98% | 96% | 96% | 126% |
| Ex9 | A-20 | B-2 (1:2) | 95% | 97% | 95% | 95% | 129% |
| Ex10 | A-83 | B-2 (1:1) | 94% | 102% | 101% | 101% | 111% |
| Ex11 | A-3 | C-7 (1:2) | 95% | 115% | 114% | 114% | 101% |
| Ex12 | A-3 | C-7 (1:1) | 93% | 112% | 111% | 111% | 100% |
| Ex13 | A-20 | C-7 (1:2) | 99% | 103% | 104% | 105% | 105% |
| Ex14 | A-83 | C-7 (1:2) | 99% | 109% | 107% | 108% | 95% |

As shown in Table 2, in comparison to the OLED of Ref2, in which the first organic layer includes the compound in Formula 7, in the OLED of Ex2 to Ex14, in which the first organic layer includes the first compound in Formula 1-1, the driving voltage is reduced, and the quantum efficiency, the blue index and the lifespan are improved.

In addition, in comparison to the OLED of Ex2, in which the second organic layer, i.e., the ETL, includes the compound in Formula 6, in the OLED of Ex3 to Ex14, in which the second organic layer includes the compound represented by one of Formulas 2-1 to 2-3, the driving voltage is reduced, and the quantum efficiency, the blue index and the lifespan are improved.

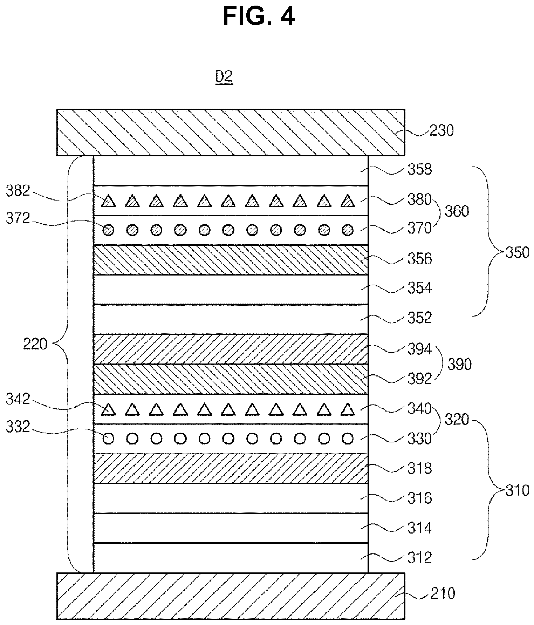

FIG. 4 is a schematic cross-sectional view of an OLED according to a third embodiment of the present disclosure.

As shown in FIG. 4, an OLED D2 includes a first electrode 210, a second electrode 230 facing the first electrode 210 and a light emitting layer 220 therebetween. The light emitting layer 220 includes a first emitting part 310 including a first EML 318 and a first intermediate organic layer 320 and a second emitting part 350 including a second EML 356 and a second intermediate organic layer 360. The second emitting part 350 is positioned between the first emitting part 310 and the second electrode 230. The first intermediate organic layer 320 is positioned between the first EML 318 and the second emitting part 350 and includes a first organic layer 330 and a second organic layer 340. The second intermediate organic layer 360 is positioned between the second EML 356 and the second electrode 230 and includes a first organic layer 370 and a second organic layer 380. In addition, the OLED D2 may further include a charge generation layer (CGL) 390 between the first and second emitting parts 310 and 350.

The organic light emitting display device 100 (of FIG. 2) includes a red pixel region, a green pixel region and a blue pixel region, and the OLED D2 is positioned in each of the red, green and blue pixel regions.

The first electrode 210 may be an anode, and the second electrode 230 may be a cathode. One of the first and second electrodes 210 and 230 is a transparent electrode (semitransparent electrode, and the other one of the first and second electrodes 210 and 230 is a reflective electrode.

The first emitting part 310 may further include at least one of a first HTL 314 between the first electrode 210 and the first EML 318 and a first EBL 316 between the first HTL 314 and the first EML 318.

In addition, the first emitting part 310 may further include an HIL 312 between the first electrode 210 and the first HTL 314.

The second emitting part 350 may further include at least one of a second HTL 352 under the second EML 356 and a second EBL 354 between the second EML 356 and the second HTL 352.

In addition, the second emitting part 350 may further include an EIL 358 between the second intermediate organic layer 360 and the second electrode 230.

Each of the first and second EMLs 318 and 356 is one of a red EML, a green EML and a blue EML. Namely, the same color light is emitted from the first and second emitting parts 310 and 350. For example, a difference between an emission wavelength range of the first emitting part 310 or the first EML 318 and an emission wavelength range of the second emitting part 350 or the second EML 356 may be 0 to 20nm.

The CGL 390 is positioned between the first and second emitting parts 310 and 350, and the first emitting part 310, the CGL 390, the second emitting part 350 are sequentially stacked on the first electrode 210. Namely, the first emitting part 310 is positioned between the first electrode 210 and the CGL 390, and the second emitting part 350 is positioned between the second electrode 230 and the CGL 390.

The first and second emitting parts 310 and 350 are connected through the CGL 390. The CGL 390 may be a P-N junction CGL of an N-type CGL 392 and a P-type CGL 394.

The N-type CGL 392 is positioned between the first intermediate organic layer 320 and the second emitting part 350, and the P-type CGL 394 is positioned between the N-type CGL 392 and the second emitting part 350. The N-type CGL 392 provides an electron to the first EML 318 of the first emitting part 310, and the P-type CGL 394 provides a hole to the second EML 356 of the second emitting part 350.

At least one of the first and second intermediate organic layers 320 and 360 includes a compound represented by Formula 1-1. For example, the first organic layer 330 of the first intermediate organic layer 320 includes a first compound 332 represented by Formula 1-1, and/or the first organic layer 370 of the second intermediate organic layer 360 includes a first compound 372 represented by Formula 1-1.

The first organic layer 330 of the first intermediate organic layer 320 may include a first compound 332 represented by Formula 1-1, and the first organic layer 370 of the second intermediate organic layer 360 may include a first compound 372 represented by Formula 1-1. In this case, the first compound 332 included in the first organic layer 330 of the first intermediate organic layer 320 and the first compound 372 included in the first organic layer 370 of the second intermediate organic layer 360 may be same or different.

The second organic layer 340 of the first intermediate organic layer 320, which includes the compound represented by Formula 1-1, and/or the second organic layer 380 of the second intermediate organic layer 360, which includes the compound represented by Formula 1-1, includes the compound represented by one of Formulas 2-1 to 2-3.

When the first organic layer 330 of the first intermediate organic layer 320 includes the first compound 332 represented by Formula 1-1, the second organic layer 340 of the first intermediate organic layer 320 includes a second compound 342 represented by one of Formulas 2-1 to 2-3. In this instance, the second organic layer 340 of the first intermediate organic layer 320 may further include a third compound (not shown) being a Li derivative. The third compound has a volume % of 10 to 200, preferably 50 to 150, with respect to the second compound 342.

When the first organic layer 370 of the second intermediate organic layer 360 includes the first compound 372 represented by Formula 1-1, the second organic layer 380 of the second intermediate organic layer 360 includes a second compound 382 represented by one of Formulas 2-1 to 2-3. In this instance, the second organic layer 380 of the second intermediate organic layer 360 may further include a third compound (not shown) being a Li derivative. The third compound has a volume % of 10 to 200, preferably 50 to 150, with respect to the second compound 382.

When the first organic layer 330 of the first intermediate organic layer 320 and the first organic layer 370 of the second intermediate organic layer 360 respectively include the first compound 332 represented by Formula 1-1 and the first compound 372 represented by Formula 1-1, the second organic layer 340 of the first intermediate organic layer 320 and the second organic layer 380 of the second intermediate organic layer 360 respectively include the second compound 342 represented by one of Formulas 2-1 to 2-3 and the second compound 382 represented by one of Formulas 2-1 to 2-3. In this instance, each of the second organic layer 340 of the first intermediate organic layer 320 and the second organic layer 380 of the second intermediate organic layer 360 may further include a third compound (not shown) being a Li derivative. In the second organic layer 340 of the first intermediate organic layer 320, the third compound has a volume % of 10 to 200, preferably 50 to 150, with respect to the second compound 342. In the second organic layer 380 of the second intermediate organic layer 360, the third compound has a volume % of 10 to 200, preferably 50 to 150, with respect to the second compound 382.

When the second organic layer 340 of the first intermediate organic layer 320 and the second organic layer 380 of the second intermediate organic layer 360 respectively include the second compound 342 represented by one of Formulas 2-1 to 2-3 and the second compound 382 represented by one of Formulas 2-1 to 2-3, the second compound 342 included in the second organic layer 340 of the first intermediate organic layer 320 and the second compound 382 included in the second organic layer 380 of the second intermediate organic layer 360 may be same or different.

In the first intermediate organic layer 320, the first organic layer 330 may have a thickness being smaller than the second organic layer 340. In the second intermediate organic layer 360, the first organic layer 370 may have a thickness being smaller than the second organic layer 380.

As illustrated above, in the OLED D2 of the present disclosure, the first emitting part 310 includes the first intermediate organic layer 320, which is disposed on the first EML 318 and includes the first and second organic layers 330 and 340, and the second emitting part 350 includes the second intermediate organic layer 360, which is disposed on the second EML 356 and includes the first and second organic layers 370 and 380. In this instance, at least one of the first organic layer 330 in the first intermediate organic layer 320 and the first organic layer 370 in the second intermediate organic layer 360 includes the first compound 332 and 372 represented by Formula 1-1. As a result, the turn-on voltage and the driving voltage of the OLED D2 are decreased so that the OLED D2 and the organic light emitting display device 100 including the OLED D2 have advantages of decrease of power consumption, increase of emitting efficiency and increase of lifespan.

In addition, since at least one of the second organic layer 340 in the first intermediate organic layer 320 and the second organic layer 380 in the second intermediate organic layer 360 includes the second compound 342 and 382 represented by one of Formulas 2-1 to 2-3, the OLED D2 and the organic light emitting display device 100 including the OLED D2 have big advantages of decrease of power consumption, increase of emitting efficiency and increase of lifespan.

Moreover, since at least one of the second organic layer 340 in the first intermediate organic layer 320 and the second organic layer 380 in the second intermediate organic layer 360 further includes the third compound being the Li derivative, the property of the OLED D2 and the organic light emitting display device 100 including the OLED D2 is further improved.

Furthermore, since the OLED D2 has a tandem structure and includes the intermediate organic layers 320 and 360, the emitting efficiency of the OLED D2 and the organic light emitting display device 100 including the OLED D2 is significantly increased with preventing or minimizing increase of the driving voltage.

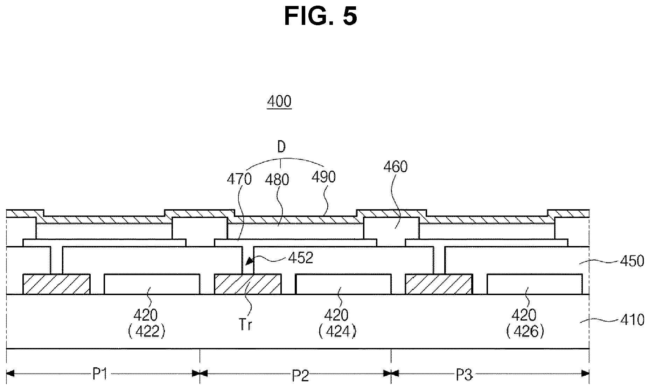

FIG. 5 is a schematic cross-sectional view of an organic light emitting display device according to a fourth embodiment of the present disclosure.

As shown in FIG. 5, an organic light emitting display device 400 includes a substrate 410, where first to third pixel regions P1, P2 and P3 are defined, a TFT Tr on or over the substrate 410, an OLED D over the TFT Tr and connected to the TFT Tr and a color filter layer 420 corresponding to the first to third pixel regions P1, P2 and P3. For example, the first pixel region P1 may be a red pixel region, the second pixel region P2 may be a green pixel region, and the third pixel region P3 may be a blue pixel region. The first to third pixel regions P1, P2 and P3 constitute a pixel unit.

The substrate 410 may be a glass substrate or a flexible substrate. For example, the flexible substrate may be one of a polyimide (PI) substrate, a polyethersulfone (PES) substrate, a polyethylenenaphthalate (PEN) substrate, a polyethylene terephthalate (PET) substrate and a polycarbonate (PC) substrate.

The TFT Tr is positioned on the substrate 410. Alternatively, a buffer layer (not shown) may be formed on the substrate 410, and the TFT Tr may be formed on the buffer layer.

As illustrated with reference to FIG. 2, the TFT Tr includes a semiconductor layer, a gate electrode, a source electrode and a drain electrode and acts as a driving element. Namely, the TFT Tr may be the driving TFT Td (of FIG. 1).

The color filter layer 420 is positioned on or over the substrate 410. For example, the color filter layer 420 may include a first color filter layer 422 corresponding to the first pixel region P1, a second color filter layer 424 corresponding to the second pixel region P2 and a third color filter layer 426 corresponding to the third pixel region P3. The first color filter layer 422 may be a red color filter layer, the second color filter layer 424 may be a green color filter layer, and the third color filter layer 426 may be a blue color filter layer. For example, the first color filter layer 422 may include at least one of red dye and red pigment, the second color filter layer 424 may include at least one of green dye and green pigment, and the third color filter layer 426 may include at least one of blue dye and blue pigment.

A planarization layer 450 is disposed on the TFT Tr and the color filter layer 420. The planarization layer 450 has a flat top surface and includes a drain contact hole 452 exposing the drain electrode of the TFT Tr.

The OLED D is disposed on the planarization layer 450 and corresponds to the color filter layer 420. The OLED D includes a first electrode 470, a light emitting layer 480 and a second electrode 490. The first electrode 470 is connected to the drain electrode of the TFT Tr through the drain contact hole 452, and the light emitting layer 480 and the second electrode 490 are sequentially stacked on the first electrode 470. The OLED D is positioned in each of the first to third pixel regions P1, P2 and P3 and emits white color light in each of the first to third pixel regions P1, P2 and P3.

The first electrode 470 may be formed to be separated in each of the first to third pixel regions P1, P2 and P3, and the second electrode 490 may be formed as one-body in correspondence to the first to third pixel regions P1, P2 and P3.

The first electrode 470 is one of an anode and a cathode, and the second electrode 490 is the other one of the anode and the cathode. The first electrode 470 is a transparent (or semitransparent) electrode, and second electrode 490 is a reflection electrode.

For example, the first electrode 470 may be an anode and may include a transparent conductive oxide material layer formed of a conductive material, e.g., a transparent conductive oxide material, having a relatively high work function. The second electrode 490 may be a cathode and may include a metallic material layer formed of a conductive material, e.g., low resistance metal, having a relatively low work function. For example, transparent conductive oxide material layer of the first electrode 470 may include one of indium-tin-oxide (ITO), indium-zinc-oxide (IZO), indium-tin-zinc-oxide (ITZO), tin oxide (SnO), zinc oxide (ZnO), indium-copper-oxide (ICO) and aluminum-zinc-oxide (Al:ZnO, AZO), and the second electrode 490 may be formed of aluminum (Al), magnesium (Mg), calcium (Ca), silver (Ag), their alloy, e.g., Mg-Ag alloy (MgAg) or their combination.

The light emitting layer 480 as an emission unit is formed on the first electrode 470. The light emitting layer 480 includes at least two emitting parts emitting different color lights. Each emitting part includes an EML and an intermediate organic layer. For example, the intermediate organic layer may have a double-layered structure including a first organic layer being an HBL and a second organic layer being an ETL. In addition, each emitting part may further include at least one of an HIL, an HTL, an EBL and an EIL. Moreover, the light emitting layer 480 may further include a CGL between emitting parts.

In this instance, at least one of the intermediate organic layers includes a first compound represented by Formula 1-1. For example, the first organic layer of the intermediate organic layer may include the first compound represented by Formula 1-1. In addition, the second organic layer of the intermediate organic layer may include a second compound represented by one of Formulas 2-1 to 2-3. Moreover, the second organic layer of the intermediate organic layer may further include a third compound being a Li derivative.

A bank layer 460, which covers an edge of the first electrode 470, is formed on the planarization layer 450. The bank layer 460 exposes a center of the first electrode 470 corresponding to the first to third pixel regions P1, P2 and P3. As illustrate above, since the OLED D emits white color light at the first to third pixel regions P1, P2 and P3, the light emitting layer 480 may be continuously formed through the first to third pixel regions P1, P2 and P3 as a common layer. The bank layer 460 is formed to prevent current leakage at an edge of the first electrode 470 and may be omitted.

The organic light emitting display device 400 may further include an encapsulation film (or an encapsulation layer) formed on the second electrode 490 to prevent penetration of moisture into the OLED D. In addition, the organic light emitting display device 400 may further include a polarization plate under the substrate 410 for reducing an ambient light reflection.

In the organic light emitting display device 400, the first electrode 470 is a transparent electrode, the second electrode 470 is a reflective electrode, and the color filter layer 420 is disposed between the substrate 410 and the OLED D. Namely, the organic light emitting display device 400 is a bottom-emission type organic light emitting display device.

Alternatively, in the organic light emitting display device 400, the first electrode 470 may be a reflective electrode, the second electrode 470 may be a transparent electrode, and the color filter layer 420 may be disposed on or over the OLED D.

In the organic light emitting display device 400, the OLED D in the first to third pixel regions P1, P2 and P3 emits white color light, and the white color light passes through the first to third color filter layers 422, 424 and 426. As a result, red, green and blue colors are respectively displayed in the first to third pixel regions P1, P2 and P3.

Although not shown, a color conversion layer may be disposed between the OLED D and the color filter layer 420. The color conversion layer may include a red, green and blue color conversion layers respectively corresponding to the first to third pixel regions P1, P2 and P3, and the white color light can be converted into red, green and blue color light by the red, green and blue color conversion layers, respectively. For example, the color conversion layer may include a quantum dot. The color purity of the organic light emitting display device 400 may be further improved by the color conversion layer.

In addition, the color conversion layer may be included instead of the color filter layer 420.

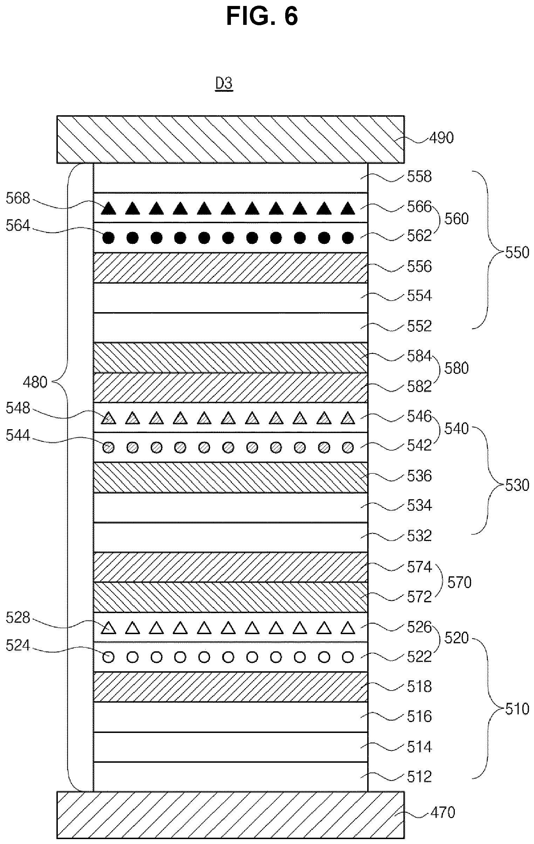

FIG. 6 is a schematic cross-sectional view of an OLED according to a fifth embodiment of the present disclosure.

As shown in FIG. 6, an OLED D3 includes a first electrode 470, a second electrode 490 facing the first electrode 470 and a light emitting layer 480 therebetween. The light emitting layer 480 includes a first emitting part 510 including a first EML 518 and a first intermediate organic layer 520, a second emitting part 530 including a second EML 536 and a second intermediate organic layer 540 and a third emitting part 550 including a third EML 556 and a third intermediate organic layer 560. The second emitting part 530 is positioned between the first emitting part 510 and the second electrode 490. The third emitting part 550 is positioned between the second emitting part 530 and the second electrode 490. The first intermediate organic layer 520 is positioned between the first EML 518 and the second emitting part 530 and includes a first organic layer 522 and a second organic layer 526. The second intermediate organic layer 540 is positioned between the second EML 536 and the third emitting part 550 and includes a first organic layer 542 and a second organic layer 546. The third intermediate organic layer 560 is positioned between the third EML 556 and the second electrode 490 and includes a first organic layer 562 and a second organic layer 566. In addition, the OLED D3 may further include a first CGL 570 between the first and second emitting parts 510 and 530 and a second CGL 580 between the second and third emitting parts 530 and 550.

The organic light emitting display device 400 (of FIG. 5) includes a red pixel region, a green pixel region and a blue pixel region, and the OLED D3 is positioned in each of the red, green and blue pixel regions.

The first electrode 470 may be an anode, and the second electrode 490 may be a cathode. One of the first and second electrodes 470 and 490 is a transparent electrode (semitransparent electrode), and the other one of the first and second electrodes 470 and 490 is a reflective electrode.

The first emitting part 510 may further include at least one of a first HTL 514 between the first electrode 470 and the first EML 518 and a first EBL 516 between the first HTL 514 and the first EML 518.

In addition, the first emitting part 510 may further include an HIL 512 between the first electrode 470 and the first HTL 514.

The second emitting part 530 may further include at least one of a second HTL 532 under the second EML 536 and a second EBL 534 between the second EML 536 and the second HTL 532.

The third emitting part 550 may further include at least one of a third HTL 552 under the third EML 556 and a third EBL 554 between the third EML 556 and the third HTL 552.

In addition, the third emitting part 550 may further include an EIL 558 between the third intermediate organic layer 560 and the second electrode 490.

One of the first to third EMLs 518, 536 and 556 is a green EML, another one of the first to third EMLs 518, 536 and 556 is a blue EML, and the other one of the first to third EMLs 518, 536 and 556 is a red EML.

For example, the first EML 518 may be a red EML, the second EML 536 may be a green EML, and the third EML 556 may be a blue EML. Alternatively, the first EML 518 may be a blue EML, the second EML 536 may be a green EML, and the third EML 556 may be a red EML

The first CGL 570 is positioned between the first and second emitting parts 510 and 530, and the second CGL 580 is positioned between the second and third emitting parts 530 and 550. Namely, the first emitting part 510, the first CGL 570, the second emitting part 530, the second CGL 580 and the third emitting part 550 are sequentially stacked on the first electrode 470. In other words, the first emitting part 510 is positioned between the first electrode 470 and the first CGL 570, and the second emitting part 530 is positioned between the first and second CGLs 570 and 580, and the third emitting part 550 is positioned between the second electrode 490 and the second CGL 580.

The first and second emitting parts 510 and 530 are connected through the first CGL 570, and the second and third emitting parts 530 and 550 are connected through the second CGL 580. The first CGL 570 may be a first P-N junction CGL of a first N-type CGL 572 and a first P-type CGL 574, and the second CGL 580 may be a second P-N junction CGL of a second N-type CGL 582 and a second P-type CGL 584.

The first N-type CGL 572 is positioned between the first intermediate organic layer 520 and the second emitting part 530, and the first P-type CGL 574 is positioned between the first N-type CGL 572 and the second emitting part 530. The first N-type CGL 572 provides an electron to the first EML 518 of the first emitting part 510, and the first P-type CGL 574 provides a hole to the second EML 536 of the second emitting part 530.

The second N-type CGL 582 is positioned between the second intermediate organic layer 540 and the third emitting part 550, and the second P-type CGL 584 is positioned between the second N-type CGL 582 and the third emitting part 550. The second N-type CGL 582 provides an electron to the second EML 536 of the second emitting part 530, and the second P-type CGL 584 provides a hole to the third EML 556 of the third emitting part 550.

At least one of the first, second and third intermediate organic layers 520, 540 and 560 includes a compound represented by Formula 1-1. For example, at least one of a first compound 524 included in the first organic layer 522 of the first intermediate organic layer 520, a first compound 544 included in the first organic layer 542 of the second intermediate organic layer 540 and a first compound 564 included in the first organic layer 562 of the third intermediate organic layer 560 is the first compound represented by Formula 1-1.

When two or more of the first compound 524 included in the first organic layer 522 of the first intermediate organic layer 520, the first compound 544 included in the first organic layer 542 of the second intermediate organic layer 540 and the first compound 564 included in the first organic layer 562 of the third intermediate organic layer 560 are the first compound represented by Formula 1-1, the first compound 524, the first compound 544 and the first compound 564 may be same or different.

The second organic layer 526 of the first intermediate organic layer 520, which includes the first compound represented by Formula 1-1, the second organic layer 546 of the second intermediate organic layer 540, which includes the first compound represented by Formula 1-1, and/or the second organic layer 566 of the third intermediate organic layer 560, which includes the first compound represented by Formula 1-1, includes the second compound represented by one of Formulas 2-1 to 2-3.

When the first organic layer 522 of the first intermediate organic layer 520 includes the first compound 524 represented by Formula 1-1, the second organic layer 526 of the first intermediate organic layer 520 includes a second compound 528 represented by one of Formulas 2-1 to 2-3. In this instance, the second organic layer 526 of the first intermediate organic layer 520 may further include a third compound (not shown) being a Li derivative. The third compound has a volume % of 10 to 200, preferably 50 to 150, with respect to the second compound 528.

When the first organic layer 542 of the second intermediate organic layer 540 includes the first compound 544 represented by Formula 1-1, the second organic layer 546 of the second intermediate organic layer 540 includes a second compound 548 represented by one of Formulas 2-1 to 2-3. In this instance, the second organic layer 546 of the second intermediate organic layer 540 may further include a third compound (not shown) being a Li derivative. The third compound has a volume % of 10 to 200, preferably 50 to 150, with respect to the second compound 548.

When the first organic layer 562 of the third intermediate organic layer 560 includes the first compound 564 represented by Formula 1-1, the second organic layer 566 of the third intermediate organic layer 560 includes a second compound 568 represented by one of Formulas 2-1 to 2-3. In this instance, the second organic layer 566 of the third intermediate organic layer 560 may further include a third compound (not shown) being a Li derivative. The third compound has a volume % of 10 to 200, preferably 50 to 150, with respect to the second compound 568.

When the first organic layer 522 of the first intermediate organic layer 520, the first organic layer 542 of the second intermediate organic layer 540 and the first organic layer 562 of the third intermediate organic layer 560 respectively include the first compound 524 represented by Formula 1-1, the first compound 544 represented by Formula 1-1 and the first compound 564 represented by Formula 1-1, the second organic layer 526 of the first intermediate organic layer 520, the second organic layer 546 of the second intermediate organic layer 540 and the second organic layer 566 of the third intermediate organic layer 560 respectively include the second compound 528 represented by one of Formulas 2-1 to 2-3, the second compound 548 represented by one of Formulas 2-1 to 2-3 and the second compound 568 represented by one of Formulas 2-1 to 2-3. In this instance, each of the second organic layer 526 of the first intermediate organic layer 520, the second organic layer 546 of the second intermediate organic layer 540 and the second organic layer 566 of the third intermediate organic layer 560 may further include a third compound (not shown) being a Li derivative. In the second organic layer 526 of the first intermediate organic layer 520, the third compound has a volume % of 10 to 200, preferably 50 to 150, with respect to the second compound 528. In the second organic layer 546 of the second intermediate organic layer 540, the third compound has a volume % of 10 to 200, preferably 50 to 150, with respect to the second compound 548. In the second organic layer 566 of the third intermediate organic layer 560, the third compound has a volume % of 10 to 200, preferably 50 to 150, with respect to the second compound 568.

When the second organic layer 526 of the first intermediate organic layer 520, the second organic layer 546 of the second intermediate organic layer 540 and the second organic layer 566 of the third intermediate organic layer 560 respectively include the second compound 528 represented by one of Formulas 2-1 to 2-3, the second compound 548 represented by one of Formulas 2-1 to 2-3 and the second compound 568 represented by one of Formulas 2-1 to 2-3, the second compound 528 included in the second organic layer 526 of the first intermediate organic layer 520, the second compound 548 included in the second organic layer 546 of the second intermediate organic layer 540 and the second compound 568 included in the second organic layer 566 of the third intermediate organic layer 560 may be same or different.

In the first intermediate organic layer 520, the first organic layer 522 may have a thickness being smaller than the second organic layer 526. In the second intermediate organic layer 540, the first organic layer 542 may have a thickness being smaller than the second organic layer 546. In the third intermediate organic layer 560, the first organic layer 562 may have a thickness being smaller than the second organic layer 566.

As illustrated above, in the OLED D3 of the present disclosure, the first emitting part 510 includes the first intermediate organic layer 520, which is disposed on the first EML 518 and includes the first and second organic layers 522 and 526, the second emitting part 530 includes the second intermediate organic layer 540, which is disposed on the second EML 536 and includes the first and second organic layers 542 and 546, and the third emitting part 550 includes the third intermediate organic layer 560, which is disposed on the third EML 556 and includes the first and second organic layers 562 and 566. In this instance, at least one of the first organic layer 522 in the first intermediate organic layer 520, the first organic layer 542 in the second intermediate organic layer 540 and the first organic layer 562 in the third intermediate organic layer 560 includes the compound represented by Formula 1-1. As a result, the turn-on voltage and the driving voltage of the OLED D3 are decreased so that the OLED D3 and the organic light emitting display device 400 including the OLED D3 have advantages of decrease of power consumption, increase of emitting efficiency and increase of lifespan.

In addition, since at least one of the second organic layer 526 in the first intermediate organic layer 520, the second organic layer 546 in the second intermediate organic layer 540 and the second organic layer 566 in the third intermediate organic layer 560 includes the compound represented by one of Formulas 2-1 to 2-3, the OLED D3 and the organic light emitting display device 400 including the OLED D3 have big advantages of decrease of power consumption, increase of emitting efficiency and increase of lifespan.

Moreover, since at least one of the second organic layer 526 in the first intermediate organic layer 520, the second organic layer 546 in the second intermediate organic layer 540 and the second organic layer 566 in the third intermediate organic layer 560 further includes the third compound being the Li derivative, the property of the OLED D3 and the organic light emitting display device 400 including the OLED D3 is further improved.

Furthermore, since the OLED D3 has a tandem structure and includes the intermediate organic layers 520, 540 and 560, the emitting efficiency of the OLED D3 and the organic light emitting display device 400 including the OLED D3 is significantly increased with preventing or minimizing increase of the driving voltage.

In the organic light emitting display device 400, the OLED D3 in the first to third pixel regions P1, P2 and P3 emits white color light, and the white color light passes through the first to third color filter layers 422, 424 and 426. As a result, red, green and blue colors are respectively displayed in the first to third pixel regions P1, P2 and P3.

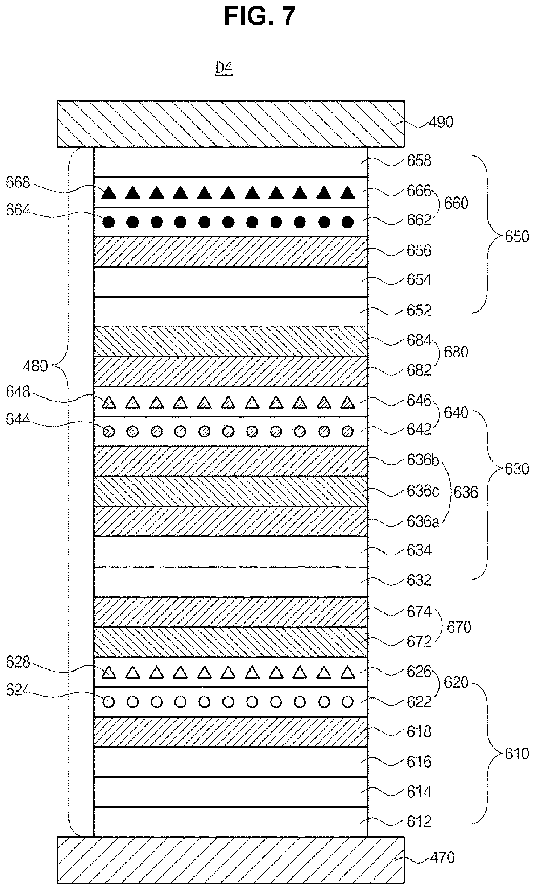

FIG. 7 is a schematic cross-sectional view of an OLED according to a sixth embodiment of the present disclosure.

As shown in FIG. 7, an OLED D4 includes a first electrode 470, a second electrode 490 facing the first electrode 470 and a light emitting layer 480 therebetween. The light emitting layer 480 includes a first emitting part 610 including a first EML 618 and a first intermediate organic layer 620, a second emitting part 630 including a second EML 636 and a second intermediate organic layer 640 and a third emitting part 650 including a third EML 656 and a third intermediate organic layer 660. The second emitting part 530 is positioned between the first emitting part 610 and the second electrode 490. The third emitting part 650 is positioned between the second emitting part 630 and the second electrode 490. The first intermediate organic layer 620 is positioned between the first EML 618 and the second emitting part 630 and includes a first organic layer 622 and a second organic layer 626. The second intermediate organic layer 640 is positioned between the second EML 636 and the third emitting part 630 and includes a first organic layer 642 and a second organic layer 644. The third intermediate organic layer 660 is positioned between the third EML 656 and the second electrode 490 and includes a first organic layer 662 and a second organic layer 664. In addition, the OLED D4 may further include a first CGL 670 between the first and second emitting parts 610 and 630 and a second CGL 680 between the second and third emitting parts 630 and 650.

The organic light emitting display device 400 (of FIG. 5) includes a red pixel region, a green pixel region and a blue pixel region, and the OLED D4 is positioned in each of the red, green and blue pixel regions.

The first electrode 470 may be an anode, and the second electrode 490 may be a cathode. One of the first and second electrodes 470 and 490 is a transparent electrode (semitransparent electrode), and the other one of the first and second electrodes 470 and 490 is a reflective electrode.

The first emitting part 610 may further include at least one of a first HTL 614 between the first electrode 470 and the first EML 618 and a first EBL 616 between the first HTL 614 and the first EML 618.

In addition, the first emitting part 610 may further include an HIL 612 between the first electrode 470 and the first HTL 614.

The second emitting part 630 may further include at least one of a second HTL 632 under the second EML 636 and a second EBL 634 between the second EML 636 and the second HTL 632.

In the second emitting part 630, the second EML 636 includes a first layer 636a, a second layer 636b over the first layer 636a and a third layer 636c between the first and second layers 636a and 636b.

The third emitting part 650 may further include at least one of a third HTL 652 under the third EML 656 and a third EBL 654 between the third EML 656 and the third HTL 552.

In addition, the third emitting part 650 may further include an EIL 658 between the second intermediate organic layer 660 and the second electrode 490.

Each of the first and third EMLs 618 and 656 is a blue EML. In the second EML 636, one of the first and second layers 636a and 636b is a red EML, and the other one of the first and second layers 636a and 636b is a green EML. The third layer 636c is a yellow-green EML. Alternatively, the third layer 636c may be omitted so that the second EML 636 may have a double-layered structure including the first and second layers 636a and 636b.

The first CGL 670 is positioned between the first and second emitting parts 610 and 630, and the second CGL 680 is positioned between the second and third emitting parts 630 and 650. Namely, the first emitting part 610, the first CGL 670, the second emitting part 630, the second CGL 680 and the third emitting part 650 are sequentially stacked on the first electrode 470. In other words, the first emitting part 610 is positioned between the first electrode 470 and the first CGL 670, and the second emitting part 630 is positioned between the first and second CGLs 670 and 680, and the third emitting part 650 is positioned between the second electrode 490 and the second CGL 680.

The first and second emitting parts 610 and 630 are connected through the first CGL 670, and the second and third emitting parts 630 and 650 are connected through the second CGL 680. The first CGL 670 may be a first P-N junction CGL of a first N-type CGL 672 and a first P-type CGL 674, and the second CGL 680 may be a second P-N junction CGL of a second N-type CGL 682 and a second P-type CGL 684.

The first N-type CGL 672 is positioned between the first intermediate organic layer 620 and the second emitting part 630, and the first P-type CGL 674 is positioned between the first N-type CGL 672 and the second emitting part 630. The first N-type CGL 672 provides an electron to the first EML 618 of the first emitting part 510, and the first P-type CGL 674 provides a hole to the second EML 636 of the second emitting part 630.

The second N-type CGL 682 is positioned between the second intermediate organic layer 640 and the third emitting part 650, and the second P-type CGL 684 is positioned between the second N-type CGL 682 and the third emitting part 650. The second N-type CGL 682 provides an electron to the second EML 636 of the second emitting part 630, and the second P-type CGL 684 provides a hole to the third EML 656 of the third emitting part 650.

At least one of the first, second and third intermediate organic layers 620, 640 and 660 includes a compound represented by Formula 1-1. For example, at least one of a first compound 624 included in the first organic layer 622 of the first intermediate organic layer 620, a first compound 644 included in the first organic layer 642 of the second intermediate organic layer 640 and a first compound 664 included in the first organic layer 662 of the third intermediate organic layer 660 is the compound represented by Formula 1-1.

When two or more of the first compound 624 included in the first organic layer 622 of the first intermediate organic layer 620, the first compound 644 included in the first organic layer 642 of the first intermediate organic layer 640 and the first compound 664 included in the first organic layer 662 of the third intermediate organic layer 660 are the compound represented by Formula 1-1, the first compound 624, the first compound 644 and the first compound 664 may be same or different.

The second organic layer 626 of the first intermediate organic layer 620, which includes the compound represented by Formula 1-1, the second organic layer 646 of the second intermediate organic layer 640, which includes the compound represented by Formula 1-1, and/or the second organic layer 666 of the third intermediate organic layer 660, which includes the compound represented by Formula 1-1, includes the compound represented by one of Formulas 2-1 to 2-3.

When the first organic layer 622 of the first intermediate organic layer 620 includes the first compound 624 represented by Formula 1-1, the second organic layer 626 of the first intermediate organic layer 620 includes a second compound 628 represented by one of Formulas 2-1 to 2-3. In this instance, the second organic layer 626 of the first intermediate organic layer 620 may further include a third compound (not shown) being a Li derivative. The third compound has a volume % of 10 to 200, preferably 50 to 150, with respect to the second compound 628.

When the first organic layer 642 of the second intermediate organic layer 640 includes the first compound 644 represented by Formula 1-1, the second organic layer 646 of the second intermediate organic layer 640 includes a second compound 648 represented by one of Formulas 2-1 to 2-3. In this instance, the second organic layer 646 of the second intermediate organic layer 640 may further include a third compound (not shown) being a Li derivative. The third compound has a volume % of 10 to 200, preferably 50 to 150, with respect to the second compound 648.

When the first organic layer 662 of the third intermediate organic layer 660 includes the first compound 664 represented by Formula 1-1, the second organic layer 666 of the third intermediate organic layer 660 includes a second compound 668 represented by one of Formulas 2-1 to 2-3. In this instance, the second organic layer 666 of the third intermediate organic layer 660 may further include a third compound (not shown) being a Li derivative. The third compound has a volume % of 10 to 200, preferably 50 to 150, with respect to the second compound 668.

When the first organic layer 622 of the first intermediate organic layer 620, the first organic layer 642 of the second intermediate organic layer 640 and the first organic layer 662 of the third intermediate organic layer 660 respectively include the first compound 624 represented by Formula 1-1, the first compound 644 represented by Formula 1-1 and the first compound 664 represented by Formula 1-1, the second organic layer 626 of the first intermediate organic layer 620, the second organic layer 646 of the second intermediate organic layer 640 and the second organic layer 666 of the third intermediate organic layer 660 respectively include the second compound 628 represented by one of Formulas 2-1 to 2-3, the second compound 648 represented by one of Formulas 2-1 to 2-3 and the second compound 668 represented by one of Formulas 2-1 to 2-3. In this instance, each of the second organic layer 626 of the first intermediate organic layer 620, the second organic layer 646 of the second intermediate organic layer 640 and the second organic layer 666 of the third intermediate organic layer 660 may further include a third compound (not shown) being a Li derivative. In the second organic layer 626 of the first intermediate organic layer 620, the third compound has a volume % of 10 to 200, preferably 50 to 150, with respect to the second compound 628. In the second organic layer 646 of the second intermediate organic layer 640, the third compound has a volume % of 10 to 200, preferably 50 to 150, with respect to the second compound 648. In the second organic layer 666 of the third intermediate organic layer 660, the third compound has a volume % of 10 to 200, preferably 50 to 150, with respect to the second compound 668.

When the second organic layer 626 of the first intermediate organic layer 620, the second organic layer 646 of the second intermediate organic layer 640 and the second organic layer 666 of the third intermediate organic layer 660 respectively include the second compound 628 represented by one of Formulas 2-1 to 2-3, the second compound 648 represented by one of Formulas 2-1 to 2-3 and the second compound 668 represented by one of Formulas 2-1 to 2-3, the second compound 628 included in the second organic layer 626 of the first intermediate organic layer 620, the second compound 648 included in the second organic layer 646 of the second intermediate organic layer 640 and the second compound 668 included in the second organic layer 666 of the third intermediate organic layer 660 may be same or different.

In the first intermediate organic layer 620, the first organic layer 622 may have a thickness being smaller than the second organic layer 626. In the second intermediate organic layer 640, the first organic layer 642 may have a thickness being smaller than the second organic layer 646. In the third intermediate organic layer 660, the first organic layer 662 may have a thickness being smaller than the second organic layer 666.

As illustrated above, in the OLED D4 of the present disclosure, the first emitting part 610 includes the first intermediate organic layer 620, which is disposed on the first EML 618 and includes the first and second organic layers 622 and 626, the second emitting part 630 includes the second intermediate organic layer 640, which is disposed on the second EML 636 and includes the first and second organic layers 642 and 646, and the third emitting part 650 includes the third intermediate organic layer 660, which is disposed on the third EML 656 and includes the first and second organic layers 662 and 666. In this instance, at least one of the first organic layer 622 in the first intermediate organic layer 620, the first organic layer 642 in the second intermediate organic layer 640 and the first organic layer 662 in the third intermediate organic layer 660 includes the first compound represented by Formula 1-1. As a result, the turn-on voltage and the driving voltage of the OLED D4 are decreased so that the OLED D4 and the organic light emitting display device 400 including the OLED D4 have advantages of decrease of power consumption, increase of emitting efficiency and increase of lifespan.

In addition, since at least one of the second organic layer 626 in the first intermediate organic layer 620, the second organic layer 646 in the second intermediate organic layer 640 and the second organic layer 666 in the third intermediate organic layer 660 includes the second compound represented by one of Formulas 2-1 to 2-3, the OLED D4 and the organic light emitting display device 400 including the OLED D4 have big advantages of decrease of power consumption, increase of emitting efficiency and increase of lifespan.

Moreover, since at least one of the second organic layer 626 in the first intermediate organic layer 620, the second organic layer 646 in the second intermediate organic layer 640 and the second organic layer 666 in the third intermediate organic layer 660 further includes the third compound being the Li derivative, the property of the OLED D4 and the organic light emitting display device 400 including the OLED D4 is further improved.

Furthermore, since the OLED D4 has a tandem structure and includes the intermediate organic layers 620, 640 and 660, the emitting efficiency of the OLED D4 and the organic light emitting display device 400 including the OLED D4 is significantly increased with preventing or minimizing increase of the driving voltage.

In the organic light emitting display device 400, the OLED D4 in the first to third pixel regions P1, P2 and P3 emits white color light, and the white color light passes through the first to third color filter layers 422, 424 and 426. As a result, red, green and blue colors are respectively displayed in the first to third pixel regions P1, P2 and P3.

In FIG. 7, the OLED D4 includes the first and third EMLs 618 and 656, each of which is a blue EML, and the second EML 636 so that the OLED D4 has a triple-layered structure. Alternatively, one of the first and third EMLs 618 and 656 may be omitted so that the OLED D4 may have a double-layered structure.

It will be apparent to those skilled in the art that various modifications and variations can be made in the present disclosure without departing from the spirit or scope of the present disclosure. Thus, it is intended that the present disclosure cover the modifications and variations of this disclosure provided they come within the scope of the appended claims and their equivalents.

The present invention refers to the following items:
[1] An organic light emitting diode, comprising:
   a first electrode;
   a second electrode facing the first electrode;
   a first emitting material layer between the first and second electrodes; and
   a first intermediate organic layer between the first emitting material layer and the second electrode and including a first organic layer and a second organic layer between the first organic layer and the second electrode,
   wherein the first organic layer includes a first compound represented by Formula 1-1: 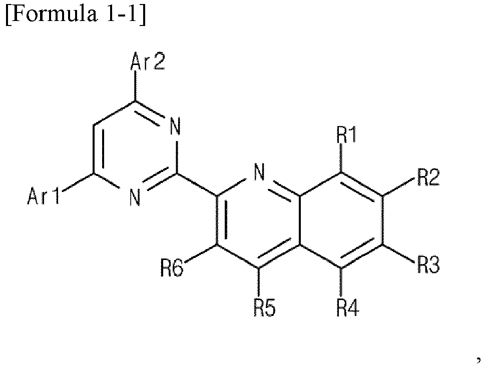
   wherein each of Ar1 and Ar2 is independently selected form the group consisting of a substituted or unsubstituted C6 to C50 aryl group and a substituted or unsubstituted C5 to C50 heteroaryl group, and
   wherein each of R1 to R6 is independently selected form the group consisting of hydrogen, deuterium, a substituted or unsubstituted C1 to C10 alkyl group, a substituted or unsubstituted C6 to C30 aryl group and a substituted or unsubstituted heteroaryl group.
[2] The organic light emitting diode according to [1], wherein the first compound is one of compounds in Formula 1-2: 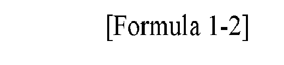 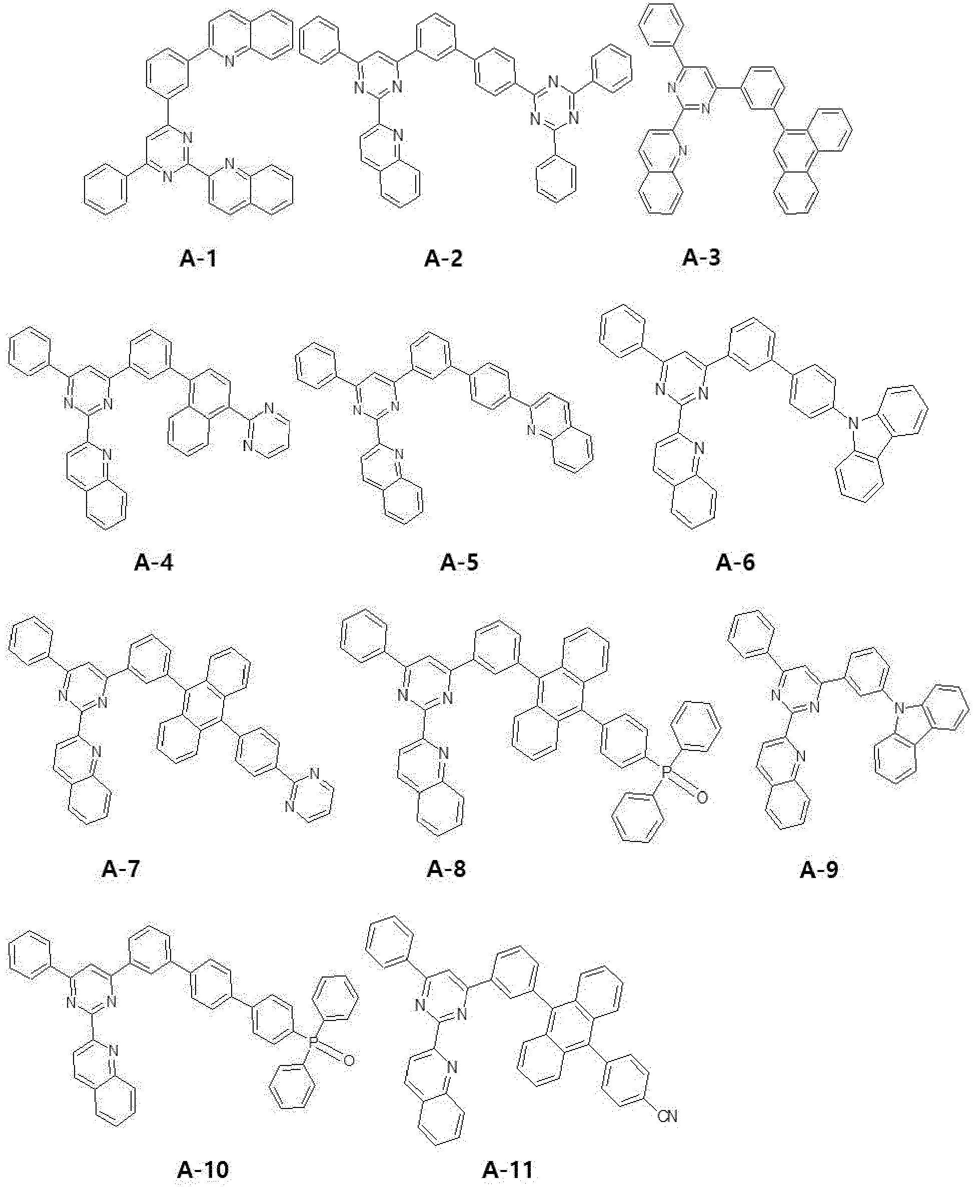 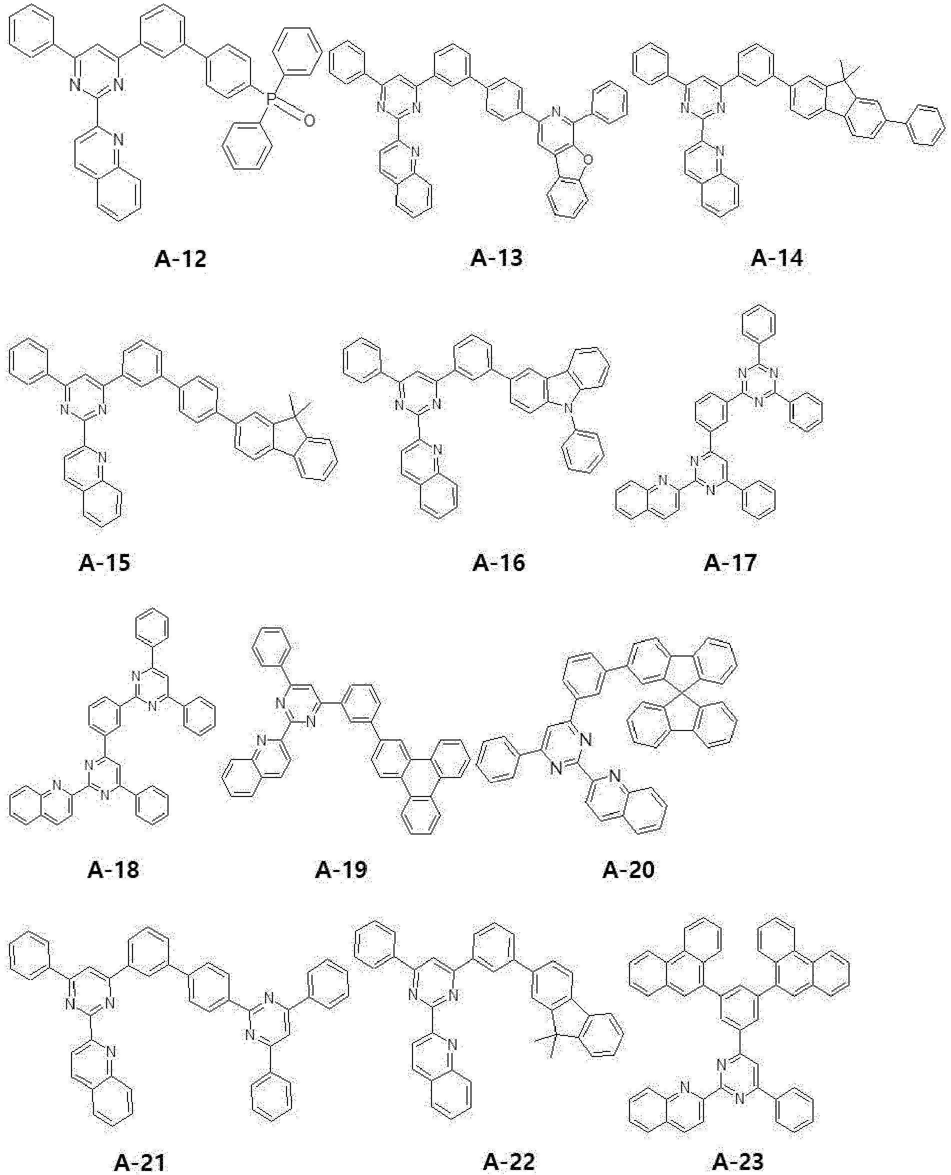 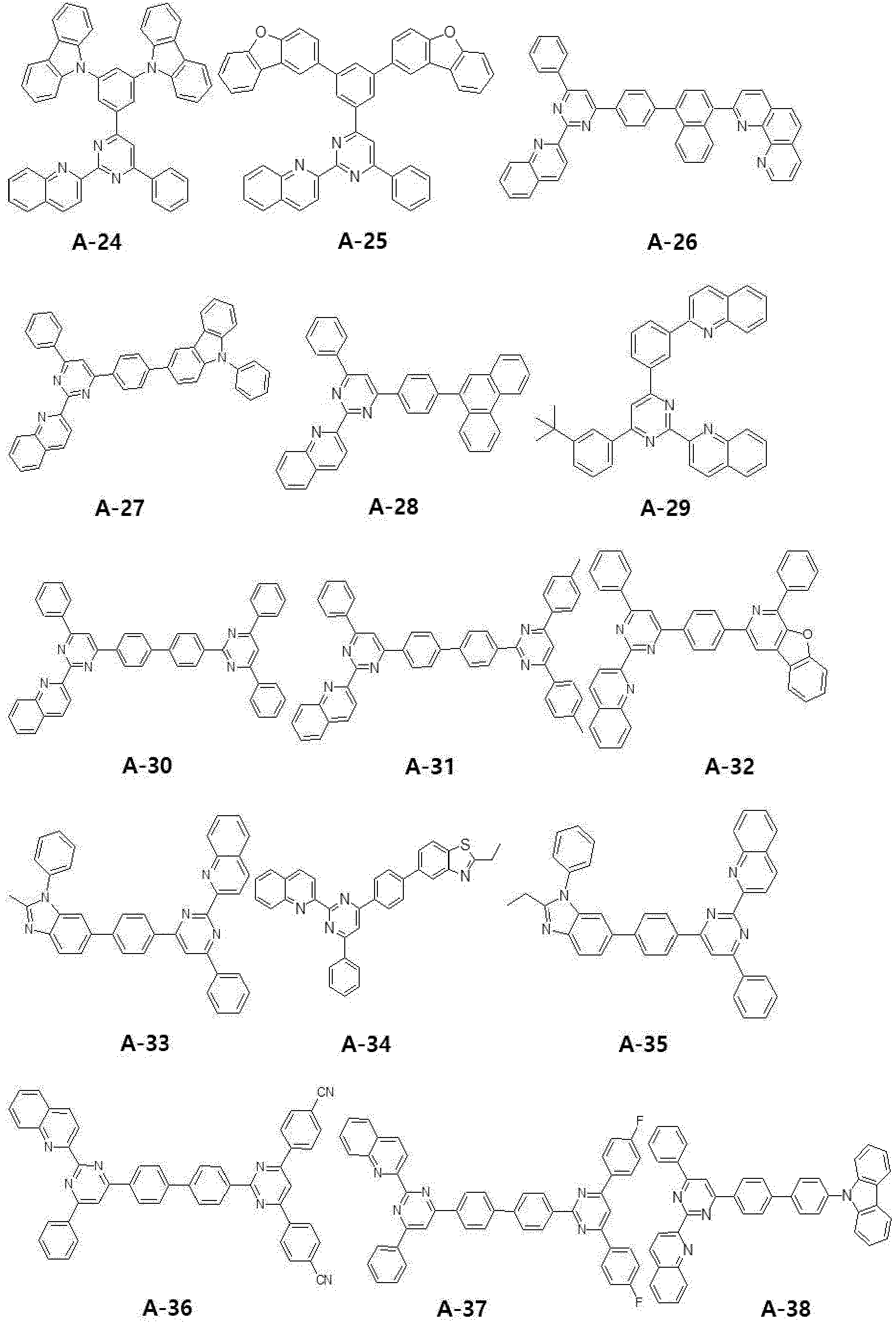 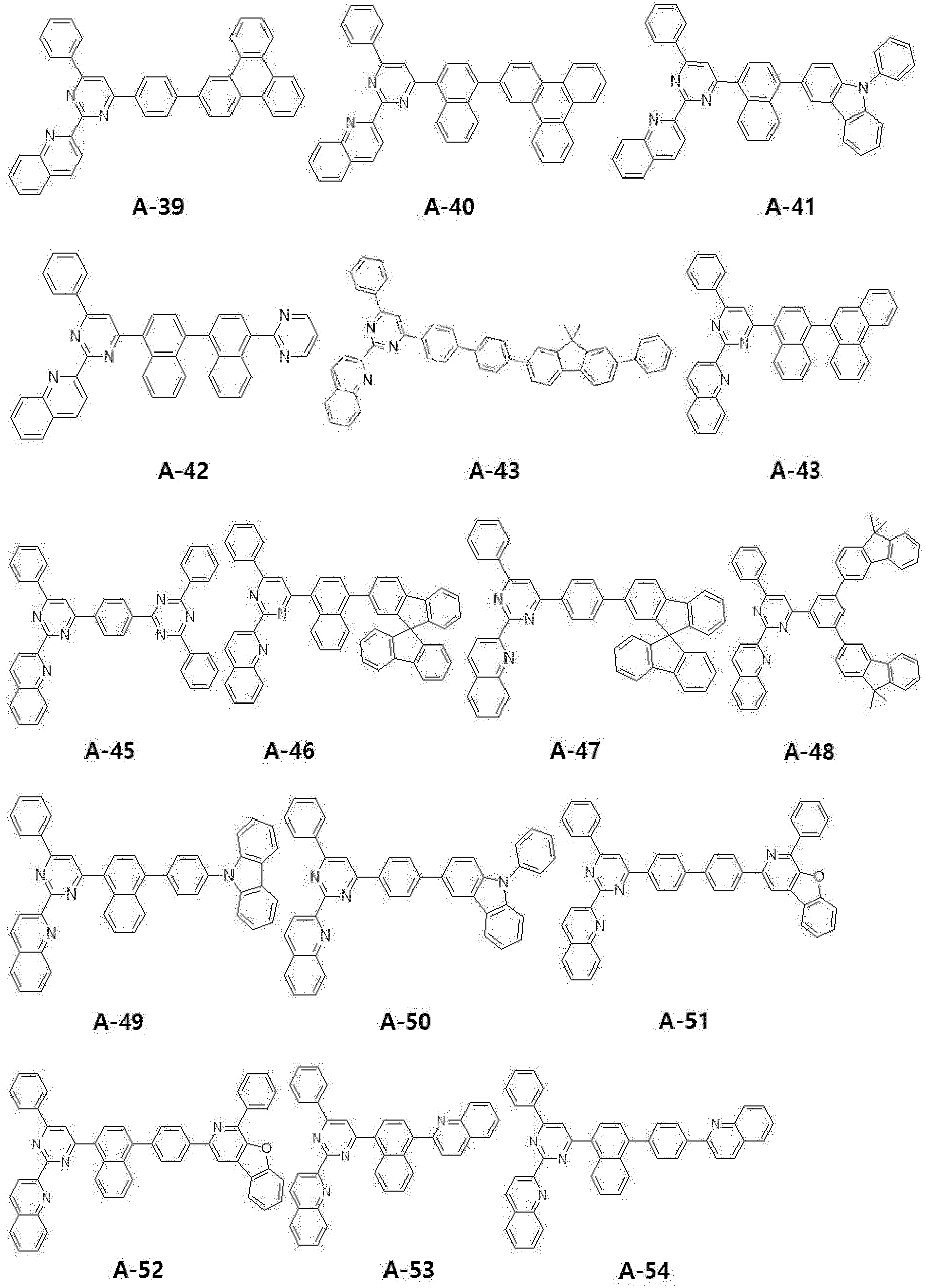 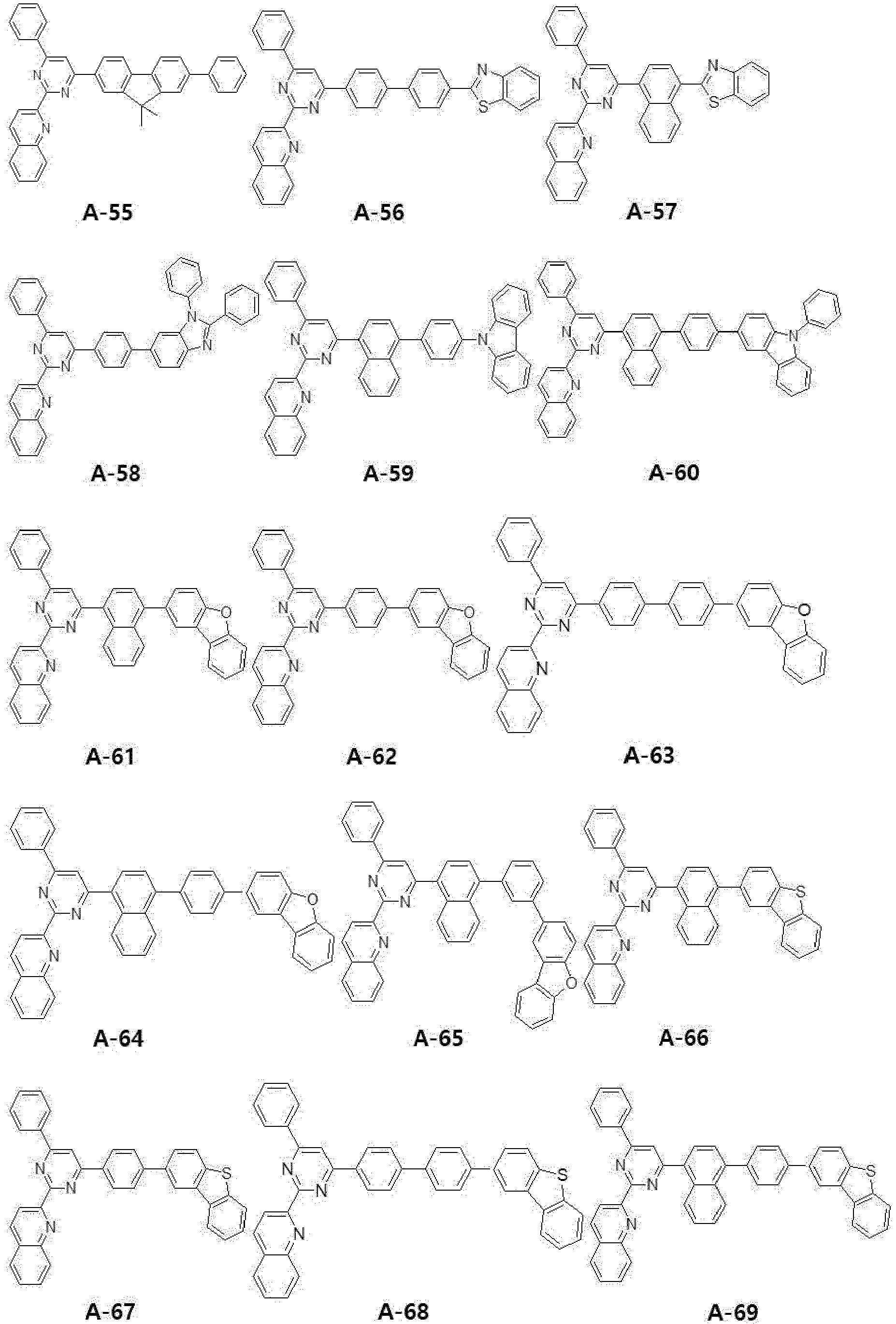 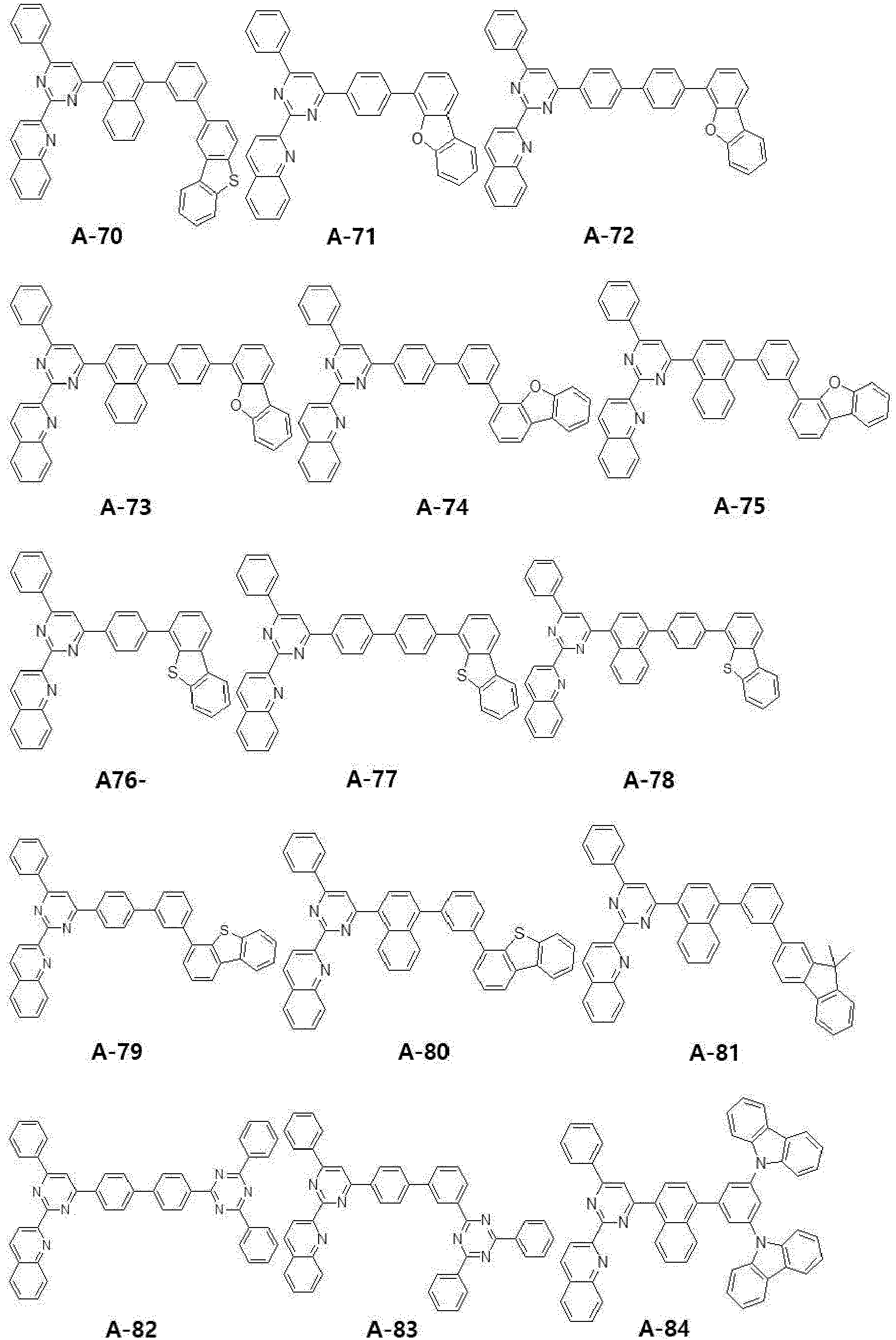 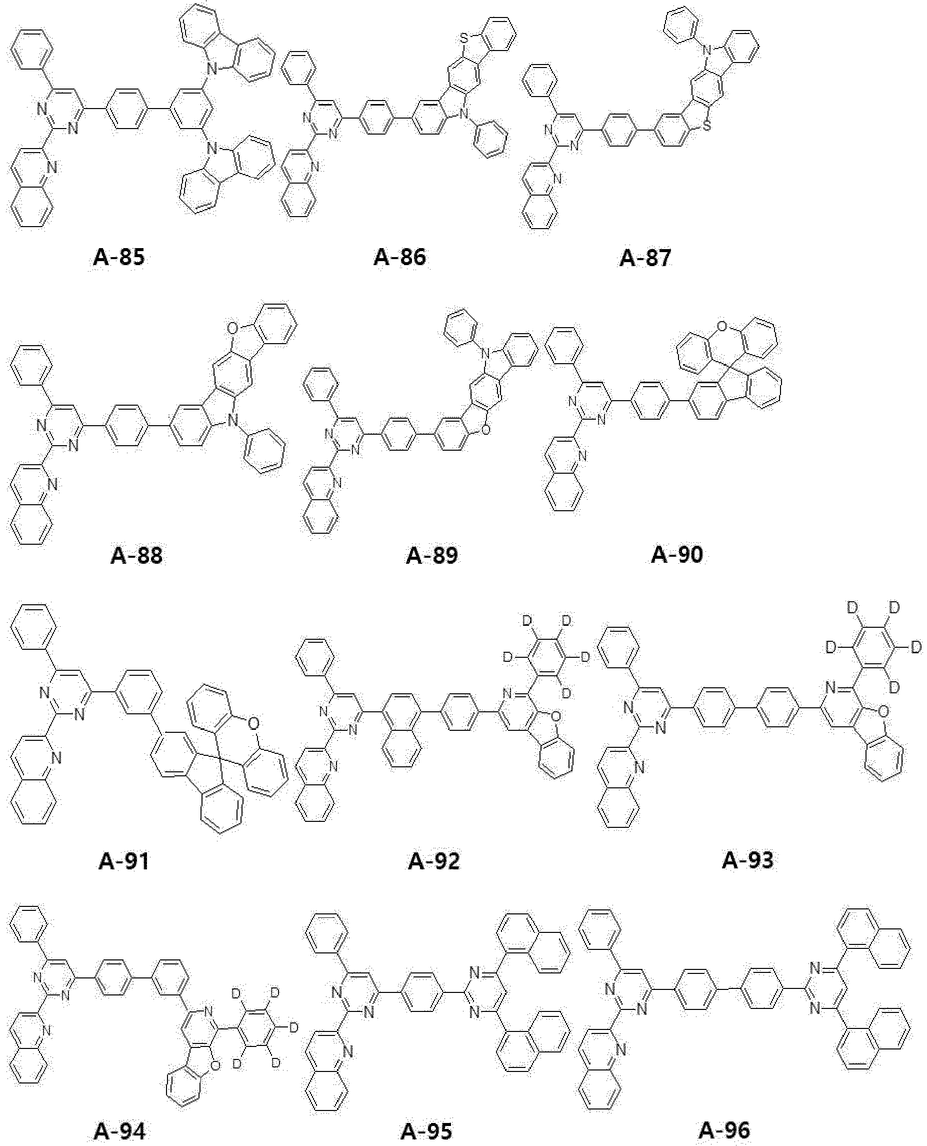 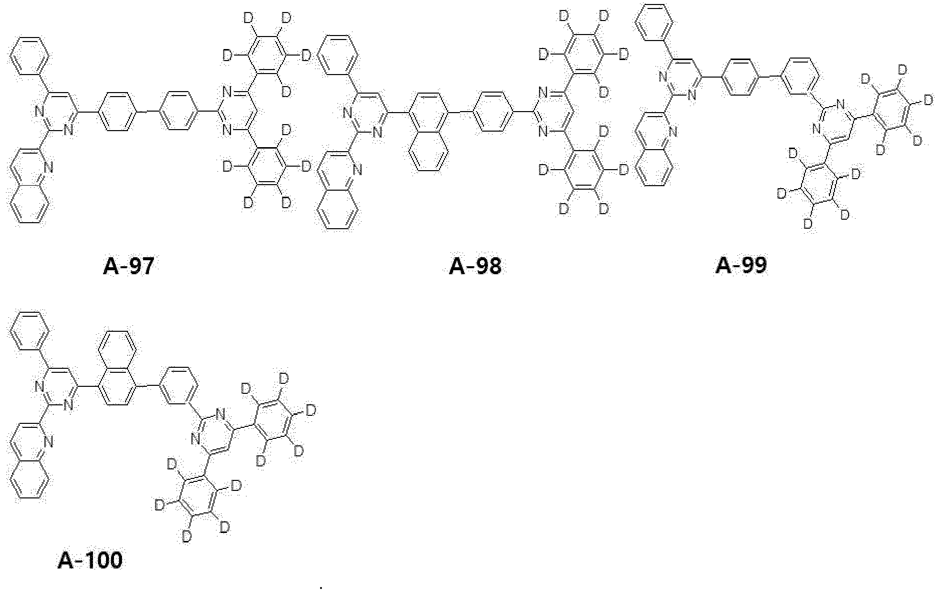
[3] The organic light emitting diode according to [1], wherein the second organic layer includes a second compound represented by one of Formulas 2-1 to 2-3: 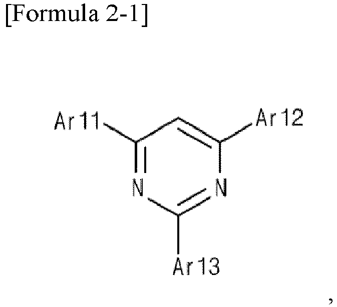 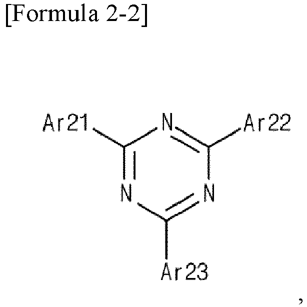 and 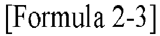 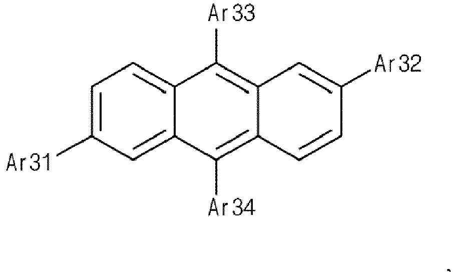
   wherein in Formula 2-1, each of Ar11 and Ar12 is independently selected from a substituted or unsubstituted C6 to C30 aryl group, and Ar13 is selected from the group consisting of a C6 to C30 aryl group substituted with a substituted or unsubstituted C5 to C30 heteroaryl group and a substituted or unsubstituted C5 to C30 heteroaryl group,
   wherein in Formula 2-2, each of Ar21 and Ar22 is independently selected from a substituted or unsubstituted C6 to C30 aryl group, and Ar23 is selected from the group consisting of a C6 to C30 aryl group unsubstituted or substituted with a C5 to C30 heteroaryl group and a substituted or unsubstituted C5 to C30 heteroaryl group, and
   wherein in Formula 2-3, each of Ar31 and Ar32 is independently selected from the group consisting of hydrogen, a substituted or unsubstituted C6 to C30 aryl group and a substituted or unsubstituted C5 to C30 heteroaryl group, and each of Ar33 and Ar34 is independently selected from the group consisting of a substituted or unsubstituted C6 to C30 aryl group and a substituted or unsubstituted C5 to C30 heteroaryl group.
[4] The organic light emitting diode according to [3], wherein the second compound is one of compounds in Formula 2-4: 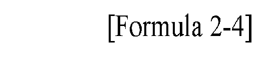 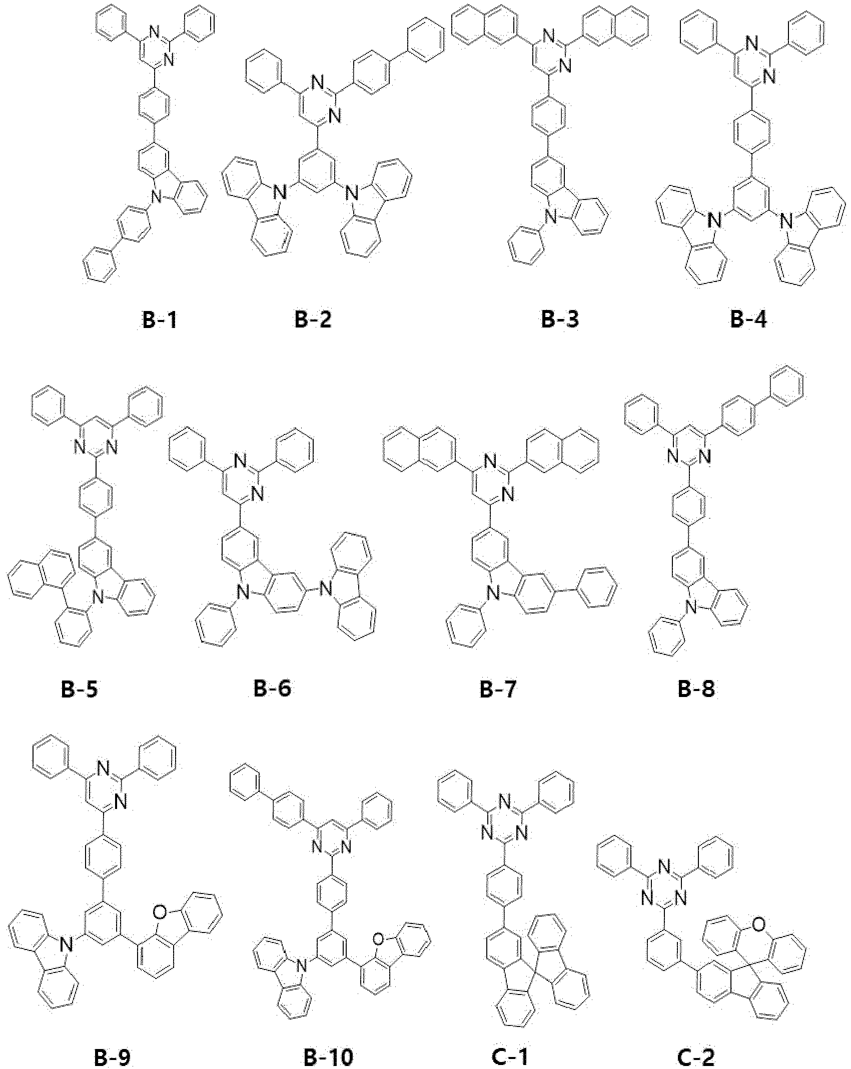 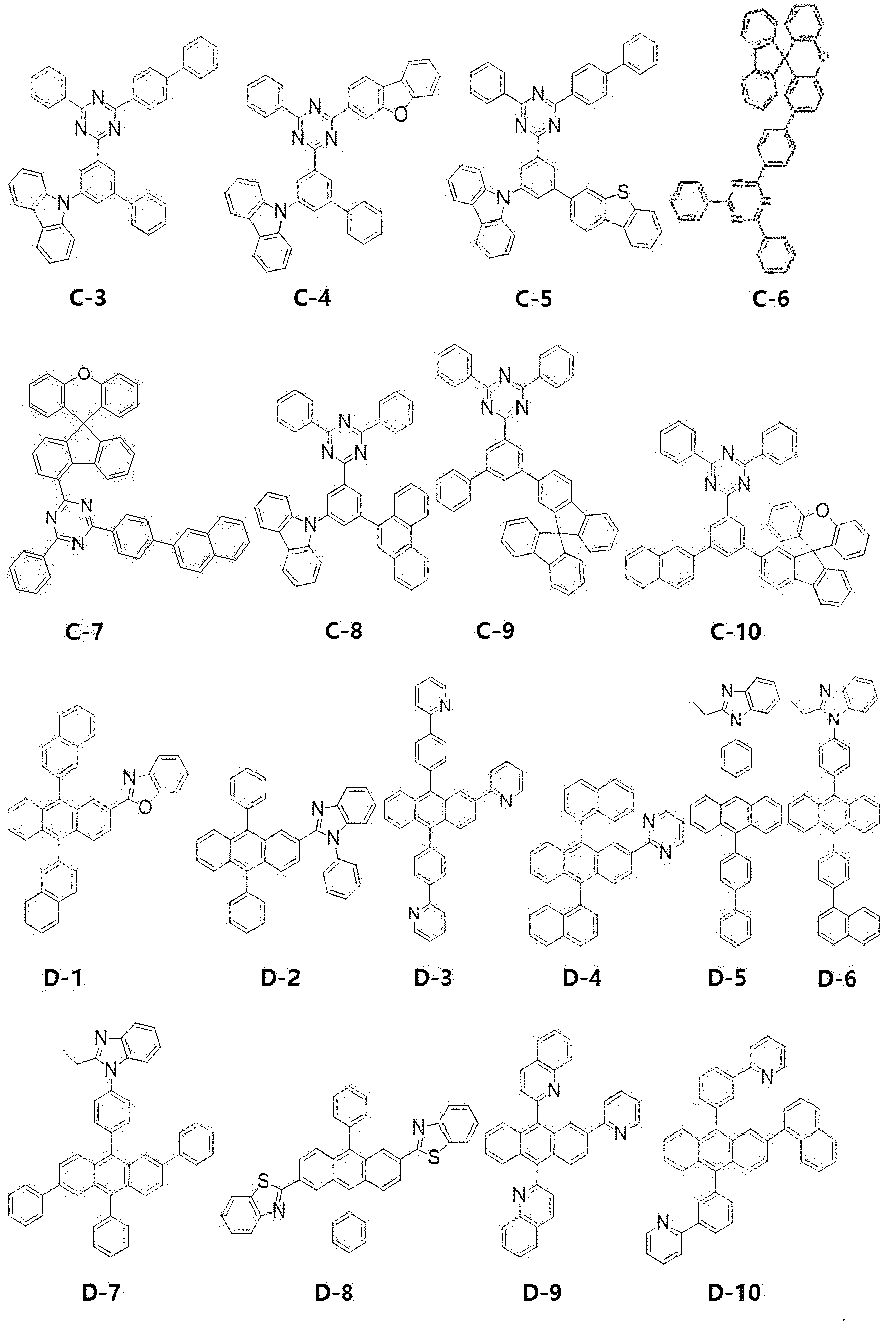
[5] The organic light emitting diode according to [3], wherein the second organic layer further includes a third compound being a Li derivative.
[6] The organic light emitting diode according to [5], wherein the third compound has a volume % of 10 to 200 with respect to the second compound.
[7] The organic light emitting diode according to [1], further comprising:
   a second emitting material layer between the first intermediate organic layer and the second electrode; and
   a second intermediate organic layer between the second emitting material layer and the second electrode and including a third organic layer and a fourth organic layer between the third organic layer and second electrode,
   wherein the third organic layer includes a fourth compound represented by Formula 1-1.
[8] The organic light emitting diode according to [7], wherein the fourth organic layer includes a fifth compound represented by one of Formulas 2-1 to 2-3.
[9] The organic light emitting diode according to [8], wherein the fourth organic layer further includes a sixth compound being a Li derivative.
[10] The organic light emitting diode according to [9], wherein the sixth compound has a volume % of 10 to 200 with respect to the fifth compound.
[11] The organic light emitting diode according to [7], wherein a difference between an emission wavelength range of the first emitting material layer and an emission wavelength range of the second emitting material layer is 0 to 20nm.
[12] The organic light emitting diode according to [7], wherein one of the first and second emitting material layers is a blue emitting material layer, and the other one of the first and second emitting material layers includes a red emitting material layer and a green emitting material layer.
[13] The organic light emitting diode according to [7], further comprising:
   a third emitting material layer between the second intermediate organic layer and the second electrode; and
   a third intermediate organic layer between the third emitting material layer and the second electrode and including a fifth organic layer and a sixth organic layer between the fifth organic layer and second electrode,
   wherein the fifth organic layer includes a seventh compound represented by Formula 1-1.
[14] The organic light emitting diode according to [13], wherein the sixth organic layer includes an eighth compound represented by one of Formulas 2-1 to 2-3.
[15] The organic light emitting diode according to [14], wherein the sixth organic layer further includes a ninth compound being a Li derivative.
[16] The organic light emitting diode according to [15], wherein the ninth compound has a volume % of 10 to 200 with respect to the eighth compound.
[17] The organic light emitting diode according to [13], wherein one of the first to third emitting material layer is a red emitting material layer, and another one of the first to third emitting material layer is a green emitting material layer, and wherein the other one of the first to third emitting material layer is a blue emitting material layer.
[18] The organic light emitting diode according to [13], wherein two of the first to third emitting material layer is a blue emitting material layer, and the other one of the first to third emitting material layer includes a red emitting material layer and a green emitting material layer.
[19] An organic light emitting device, comprising:
   a substrate; and
   an organic light emitting diode positioned over the substrate, the organic light emitting diode including:
      a first electrode;
      a second electrode facing the first electrode;
      a first emitting material layer between the first and second electrodes; and
      a first intermediate organic layer between the first emitting material layer and the second electrode and including a first organic layer and a second organic layer between the first organic layer and the second electrode,
   wherein the first organic layer includes a first compound represented by Formula 1-1: 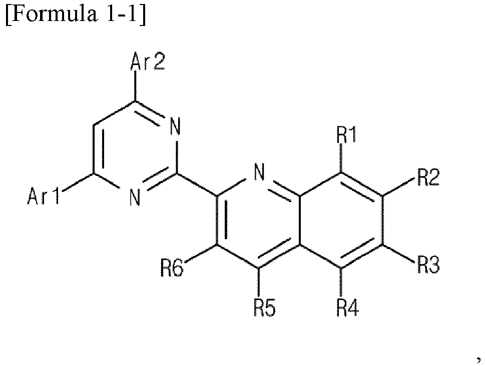
      wherein each of Ar1 and Ar2 is independently selected form the group consisting of a substituted or unsubstituted C6 to C50 aryl group and a substituted or unsubstituted C5 to C50 heteroaryl group, and
      wherein each of R1 to R6 is independently selected form the group consisting of hydrogen, deuterium, a substituted or unsubstituted C1 to C10 alkyl group, a substituted or unsubstituted C6 to C30 aryl group and a substituted or unsubstituted heteroaryl group.
[20] The organic light emitting device according to [19], further comprising: a color filter layer between the substrate and the organic light emitting diode or over the organic light emitting diode,
   wherein a red pixel region, a green pixel region and a blue pixel region are defined on the substrate,
   wherein the organic light emitting diode corresponds to each of the red, green and blue pixel region, and
   wherein the color filter layer corresponds to each of the red, green and blue pixel region.

## Claims

1. An organic light emitting diode, comprising:
a first electrode;
a second electrode facing the first electrode;
a first emitting material layer between the first and second electrodes; and
a first intermediate organic layer between the first emitting material layer and the second electrode and including a first organic layer and a second organic layer between the first organic layer and the second electrode,
wherein the first organic layer includes a first compound represented by Formula 1-1: 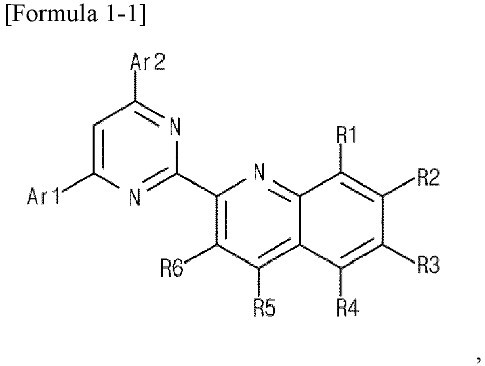
wherein each of Ar1 and Ar2 is independently selected from the group consisting of a substituted or unsubstituted C6 to C50 aryl group and a substituted or unsubstituted C5 to C50 heteroaryl group, and
wherein each of R1 to R6 is independently selected from the group consisting of hydrogen, deuterium, a substituted or unsubstituted C1 to C10 alkyl group, a substituted or unsubstituted C6 to C30 aryl group and a substituted or unsubstituted C5 to C30 heteroaryl group.

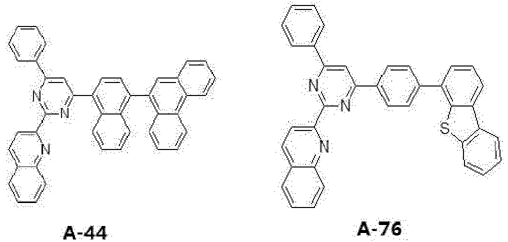

2. The organic light emitting diode according to claim 1, wherein the second organic layer includes a second compound represented by one of Formulas 2-1 to 2-3: 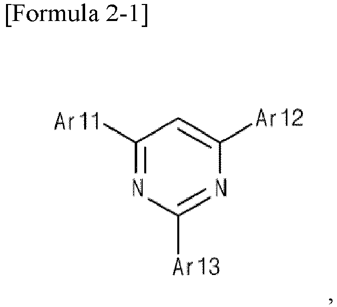 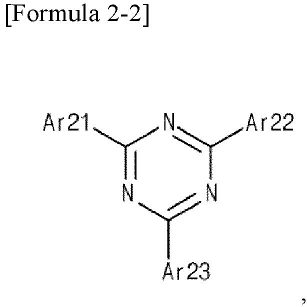 and 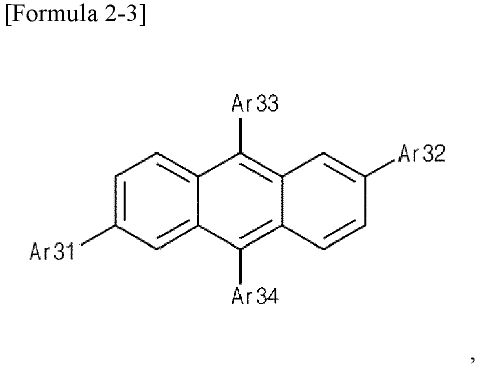
wherein in Formula 2-1, two of Ar11 to Ar13 are independently selected from a substituted or unsubstituted C6 to C30 aryl group, and the other one of Ar11 to Ar13 is selected from the group consisting of a C6 to C30 aryl group substituted with a substituted or unsubstituted C5 to C30 heteroaryl group and a substituted or unsubstituted C5 to C30 heteroaryl group,
wherein in Formula 2-2, each of Ar21 and Ar22 is independently selected from a substituted or unsubstituted C6 to C30 aryl group, and Ar23 is selected from the group consisting of a C6 to C30 aryl group unsubstituted or substituted with a C5 to C30 heteroaryl group and a substituted or unsubstituted C5 to C30 heteroaryl group, and
wherein in Formula 2-3, each of Ar31 and Ar32 is independently selected from the group consisting of hydrogen, a substituted or unsubstituted C6 to C30 aryl group and a substituted or unsubstituted C5 to C30 heteroaryl group, and each of Ar33 and Ar34 is independently selected from the group consisting of a substituted or unsubstituted C6 to C30 aryl group and a substituted or unsubstituted C5 to C30 heteroaryl group.

3. The organic light emitting diode according to claim 1 or 2, wherein the second organic layer further includes a third compound being a Li derivative.

4. The organic light emitting diode according to claim 3, wherein the third compound has a volume % of 10 to 200 with respect to the second compound.

5. The organic light emitting diode according to anyone of claims 1 to 4, further comprising:
a second emitting material layer between the first intermediate organic layer and the second electrode; and
a second intermediate organic layer between the second emitting material layer and the second electrode and including a third organic layer and a fourth organic layer between the third organic layer and second electrode,
wherein the third organic layer includes a fourth compound represented by Formula 1-1.

6. The organic light emitting diode according to claim 5, wherein the fourth organic layer includes a fifth compound represented by one of Formulas 2-1 to 2-3.

7. The organic light emitting diode according to claim 5, wherein a difference between an emission wavelength range of the first emitting material layer and an emission wavelength range of the second emitting material layer is 0 to 20nm.

8. The organic light emitting diode according to claim 5, further comprising:
a third emitting material layer between the second intermediate organic layer and the second electrode; and
a third intermediate organic layer between the third emitting material layer and the second electrode and including a fifth organic layer and a sixth organic layer between the fifth organic layer and second electrode,
wherein the fifth organic layer includes a seventh compound represented by Formula 1-1.

9. The organic light emitting diode according to claim 8, wherein the sixth organic layer includes an eighth compound represented by one of Formulas 2-1 to 2-3.

10. The organic light emitting diode according to claim 9, wherein the sixth organic layer further includes a ninth compound being a Li derivative.

11. The organic light emitting diode according to claim 10, wherein the ninth compound has a volume % of 10 to 200 with respect to the eighth compound.

12. The organic light emitting diode according to anyone of claims 8-11, wherein one of the first to third emitting material layer is a red emitting material layer, and another one of the first to third emitting material layer is a green emitting material layer, and wherein the other one of the first to third emitting material layer is a blue emitting material layer.

13. The organic light emitting diode according to claim 8-11, wherein two of the first to third emitting material layer is a blue emitting material layer, and the other one of the first to third emitting material layer includes a red emitting material layer and a green emitting material layer.

14. An organic light emitting device, comprising:
a substrate; and
an organic light emitting diode positioned over the substrate, wherein the organic light emitting diode is according to any one of claims 1 to 13.

15. The organic light emitting device according to claim 14, further comprising: a color filter layer between the substrate and the organic light emitting diode or over the organic light emitting diode,
wherein a red pixel region, a green pixel region and a blue pixel region are defined on the substrate,
wherein the organic light emitting diode corresponds to each of the red, green and blue pixel region, and
wherein the color filter layer corresponds to each of the red, green and blue pixel region.
